# EUROPÄISCHE PATENTSCHRIFT

(11) **EP 3 463 582 B1**
(45) Veröffentlichungstag und Bekanntmachung des Hinweises auf die Patenterteilung: **30.12.2020**
(21) Anmeldenummer: 17721144.8
(22) Anmeldetag: 03.05.2017
(51) Int. Cl.: A61Q 5/08, A61Q 5/10, A61K 8/362, A61K 8/44, A61K 8/49, A61K 8/22, A61K 8/23, A61K 8/02

(54) **BLONDIERMITTEL UND VERFAHREN ZUR SCHONENDEN OXIDATIVEN HAARAUFHELLUNG I**
BLEACHING AGENT AND METHOD FOR GENTLE OXIDATIVE HAIR LIGHTENING I
PRODUIT DÉCOLORANT ET PROCÉDÉ D'ÉCLAIRCISSEMENT PAR OXYDATION DOUX POUR LES CHEVEUX I

(30) Priorität: 31.05.2016 DE 102016209471
(43) Veröffentlichungstag der Anmeldung: 10.04.2019
(73) Patentinhaber: Henkel AG & Co. KGaA, 40589 Düsseldorf (DE)
(72) Erfinder: ANDERHEGGEN, Bernd, 41189 Mönchengladbach (DE); MÜLLER, Burkhard, 40221 Düsseldorf (DE)
(86) Internationale Anmeldenummer: PCT/EP2017/060535
(87) Internationale Veröffentlichungsnummer: WO 2017/207198

(56) Entgegenhaltungen:
- DE-A1- 10 051 774
- DE-A1-102005 053 850
- E Oshimura ET AL: "Effects of arginine on hair damage via oxidative coloring process.", Journal of Cosmetic Science Supplement, Bd. Supplement 1. Januar 2004 (2004-01-01), Seiten 155-170, XP055388284, Gefunden im Internet: URL:http://onlinelibrary.wiley.com/store/1 0.1111/j.1467-2494.2005.00257_13.x/asset/j .1467-2494.2005.00257_13.x.pdf?v=1&t=j4r4a 5bv&s=0234c330a5f96451e4e7d42d8040ad5a44f6 7dec [gefunden am 2017-07-05]

## Beschreibung

Die vorliegende Erfindung betrifft Blondierpulver, die als Mittel zum Aufhellen von keratinischen Fasern, insbesondere menschlichen Haaren, dienen. Weiterhin betrifft die vorliegende Erfindung die Verwendung der Mittel zur schonenden Blondierung bzw. oxidativen Aufhellung von menschlichen Haaren sowie eine Mehrkomponentenverpackungseinheit (Kit-of-Parts) zur Aufhellung keratinischer Fasern, die ein Blondierpulver und davon getrennt eine Oxidationsmittelzubereitung umfasst. Weiterhin wird ein Verfahren zur oxidativen Aufhellung von keratinischen Fasern unter Verwendung des genannten Blondierpulvers und des dieses Blondierpulver umfassenden Kits beschrieben. Bei dem Blondierpulver handelt es sich eine wasserfreie, pulverförmige Oxidationsmittelzubereitung, enthaltend mindestens ein Persalz oder Percarbonat.

Das Aufhellen der eigenen Haarfarbe ist seit jeher der Wunsch vieler Verbraucher, da eine blonde Haarfarbe als attraktiv und in modischer Hinsicht erstrebenswert betrachtet wird. Für diesen Zweck sind im Markt verschiedene Blondiermittel mit unterschiedlicher Blondierleistung erhältlich. Die in diesen Produkten enthaltenen Oxidationsmittel sind in der Lage, durch die oxidative Zerstörung des haareigenen Farbstoffes Melanin die Haarfaser aufzuhellen. Für einen moderaten Blondiereffekt genügt der Einsatz von Wasserstoffperoxid - gegebenenfalls unter Einsatz von Ammoniak oder anderen Alkalisierungsmitteln - als Oxidationsmittel allein. Für das Erzielen eines stärkeren Blondiereffekts wird üblicherweise eine Mischung aus Wasserstoffperoxid und mindestens einer Verbindung, ausgewählt aus Percarbonaten und Persalzen, insbesondere Peroxodisulfatsalzen und/oder Peroxomonosulfatsalzen, eingesetzt. Zur Verstärkung der Blondierwirkung enthalten die Mittel höhere Einsatzkonzentrationen an Wasserstoffperoxid und Percarbonaten oder Persalzen, insbesondere Persulfaten. Dunkles, dunkelbraunes oder schwarzes Haar lässt sich so in einem Schritt um 4 bis 6 Nuancen aufhellen. Das Wasserstoffperoxid und die Percarbonate oder Persalze werden bis zur Anwendung getrennt voneinander aufbewahrt, um die Percarbonate oder Persalze nicht vorzeitig zu deaktivieren. Die Wasserstoffperoxid-Komponente, die eine wässrige Lösung von Wasserstoffperoxid umfasst, weist zur Stabilisierung des Wasserstoffperoxids einen sauren pH-Wert, insbesondere einen pH-Wert von 2,5 bis 5,5, insbesondere von 3 bis 5, auf, jeweils gemessen bei 20°C.
Fürdie Melanin abbauende Wirkung des Wasserstoffperoxids und die Blondierwirkung auf der keratinischen Faser ist es jedoch vorteilhaft, wenn die Anwendungsmischung aus WasserstoffperoxidLösung und Persalz einen alkalischen pH-Wert besitzt, der vorzugsweise im Bereich von 8 bis 12, besonders bevorzugt im Bereich von 8,5 bis 11,5, außerordentlich bevorzugt im Bereich von 9 bis 10,5 liegt, jeweils gemessen bei 20°C.
Zur Einstellung eines alkalischen pH-Werts der aufhellenden Anwendungsmischung gibt es mehrere Möglichkeiten:
Das Blondierpulver enthält neben dem mindestens einen Persalz oder Percarbonat mindestens ein pulverförmiges Alkalisierungsmittel in einer solchen Gesamtmenge, dass die Anwendungsmischung den gewünschten alkalischen pH-Wert aufweist; oder
die Wasserstoffperoxidlösung wird nicht nur mit dem Blondierpulver, sondern zusätzlich mit einer Alkalisierungsmittelzubereitung zur Anwendungsmischung kombiniert.
Setzt man der Alkalisierungsmittelzubereitung und/oder dem Blondierpulver Oxidationsfarbstoffvorprodukte und/oder direktziehende Farbstoffe zu, so kann das Haar gleichzeitig gefärbt werden. Entsprechende 3-Komponenten-Haarfärbemittel werden insbesondere für Verbraucher mit sehr dunklem Melanin-reichen Haar angeboten.
Mit der Aufhellung geht jedoch auch eine Schädigung des Haares einher, da nicht nur die Farbstoffe des Haares, sondern auch die Strukturbestandteile des Haares oxidativ geschädigt werden. Je nach Ausprägung des Schädigungsgrades reicht dieser von rauem, sprödem und schwieriger auskämmbarem Haar über eine verminderte Widerstandsfähigkeit und Reißfestigkeit des Haares bis hin zu Haarbruch. Je größer die Menge des eingesetzten Wasserstoffperoxids und gegebenenfalls der Persalze oder Percarbonate ist, desto stärkere Schädigungen werden daher in der Regel auf den Keratinfasern hervorgerufen.
Um die Haarschädigung zu minimieren und den schädigenden Effekt der Oxidationsmittel auszugleichen, wird immer wieder versucht, Persalz-haltige Haaraufhellungs- und -färbemittel mit einem höheren Gehalt an Ölen zu formulieren.
Im Stand der Technik sind beispielsweise Blondiermittelsuspensionen beschrieben, die wasserfreie Suspensionen von feinteiligen, bei 25°C und 1013 mbar festen Persalzen oder Percarbonaten in einem Öl oder einer Ölmischung darstellen, die gegebenenfalls mit einem Ölgeliermittel verdickt sein kann, siehe EP 0778020, EP 1034777 und EP 1380287. Nachteilig hierbei ist, dass das Herstellen einer homogenen Mischung aus dieser sehr hydrophoben Paste und der stark wasserhaltigen Wasserstoffperoxidzubereitung sowie ggf. der üblicherweise ebenfalls wasserhaltigen Alkalisierungsmittelzubereitung schwierig ist und länger andauerndes, kräftiges Schütteln oder Rühren erfordert. Auch ist die Herstellung einer Blondiermittelsuspension technisch aufwändiger als die Herstellung einer pulverförmigen Persalzmischung.
Es war daher die Aufgabe der vorliegenden Erfindung, Mittel zum Aufhellen bzw. Blondieren von keratinischen Fasern, insbesondere menschlichen Haaren, bereitzustellen, die die Keratinfasern möglichst wenig schädigen und die einfach herzustellen und zu handhaben sind. Die Verringerung der Faserschädigung sollte dabei möglichst nicht durch Öle, sondern durch alternative Pflegewirkstoffe erzielt werden.
Überraschend wurden diese Aufgaben durch den Gegenstand der Ansprüche gelöst.
Unter keratinhaltigen bzw. keratinischen Fasern werden erfindungsgemäß Pelze, Wolle, Federn und insbesondere menschliche Haare verstanden. Obwohl die erfindungsgemäßen Mittel in erster Linie zum Blondieren und/oder Aufhellen von keratinhaltigen Fasern geeignet sind, steht prinzipiell einer Verwendung auch auf anderen Gebieten nichts entgegen.
Ein geeigneter Parameter für die Quantifizierung der Faserschädigung, insbesondere der Haarschädigung, ist die Messung der Zugfestigkeit (Young-Modul) der Keratinfasern.
WO 2005/115314A1 offenbart ein Verfahren zur Restrukturierung keratinischer Fasern, bei dem die Keratinfasern mit Cystin und mit mindestens einer Dicarbonsäure mit 2 bis 10 Kohlenstoffatomen in Kontakt gebracht werden, wobei bevorzugte Dicarbonsäuren ausgewählt sind aus Oxalsäure, Malonsäure, Bernsteinsäure, Glutarsäure, Adipinsäure, Pimelinsäure, Azelainsäure, Maleinsäure, Fumarsäure und Sorbinsäure und Bernsteinsäure besonders bevorzugt ist. DE 10051774 A1 beschreibt die Verwendung kurzkettiger Carbonsäuren mit einem Molekulargewicht unter 750 g/mol in kosmetischen Mitteln als Wirkstoff zur Restrukturierung keratinischer Fasern. EP1174112A offenbart Haarbehandlungsmittel, die neben einer organischen Säure als weitere zwingende Bestandteile ein organisches Lösungsmittel, ein kationisches Tensid und einen höheren Alkohol enthalten und zur Reparatur von Poren in Haaren dienen. DE102005053850A1 offenbart wasserfreie Blondierpulver und Blondierpasten mit einer festen Peroxoverbindung, einem Alkalisierungsmittel, einem nicht hydroxylierten Fettsäureester mit einem Schmelzpunkt von höchstens 50°C und einer C₁₀- bis C₃₀-Fettsäure mit mindestens einer zusätzlichen Hydroxygruppe, die Lysin als haarstrukturverbessernden Wirkstoff enthalten können.

Ein erster Gegenstand der vorliegenden Erfindung ist ein Blondierpulver, enthaltend
a. mindestens ein Oxidationsmittel, ausgewählt aus Natriumpercarbonaten und anorganischen Salzen einer Peroxoschwefelsäure, und sowie Mischungen hiervon,
b. weiterhin mindestens eine Dicarbonsäure mit 2 bis 10 Kohlenstoffatomen, ausgewählt aus Bernsteinsäure und/oder mindestens einem Salz dieser Säure sowie Mischungen dieser Verbindungen,
c. weiterhin Arginin und Lysin oder mindestens eines der Salze dieser Aminosäuren,
d. und 0 bis 8 Gew.-% Wasser, bezogen auf das Gewicht des Blondierpulvers.

Unter dem Begriff "Pulver" oder "pulverförmig" ist erfindungsgemäß eine bei 20 °C und 1013 mbar feste, rieselfähige Darreichungsform aus einzelnen Partikeln zu verstehen, bei der die einzelnen Partikel Partikelgrößen im Bereich von 0,1 µm bis maximal 1,6 mm aufweisen. Die Bestimmung der Partikelgrößen kann bevorzugt mittels Laserbeugungsmessung gemäß ISO 13320-1 (2009) erfolgen. Gegebenenfalls können die Partikel durch physikalische Behandlung, wie Sieben, Pressen, Granulieren oder Pelletieren, oder durch den Zusatz bestimmter Hilfsstoffe in ihrer Korngröße den Anforderungen an das Blondierpulver angepasst werden, um beispielsweise eine bessere Mischbarkeit der einzelnen Pulverbestandteile oder die Mischbarkeit des Blondierpulvers mit einer Wasserstoffperoxid-Zubereitung zu ermöglichen.
Erfindungsgemäß bevorzugte Blondierpulver weisen eine Schüttdichte im Bereich von 500 bis 1000 g/l (Gramm/Liter), bevorzugt 550 bis 900 g/l, besonders bevorzugt 600 bis 820 g/l, auf. Die Bestimmung der Schüttdichte erfolgt bevorzugt nach EN ISO 600 DIN 53468.
Sofern nicht anders angegeben, beziehen sich alle Temperaturangaben auf einen Druck von 1013 mbar.
Das erfindungsgemäße Blondierpulver enthält als ersten wesentlichen Bestandteil mindestens ein Oxidationsmittel, das aus Natriumpercarbonaten und anorganischen Salzen einer Peroxoschwefelsäure sowie Mischungen hiervon ausgewählt ist.
Unter Natriumpercarbonaten werden Natriumcarbonat-Wasserstoffperoxid-Komplexe verstanden. Handelsübliches Natriumpercarbonat hat die durchschnittliche Zusammensetzung 2 Na₂CO₃ · 3 H₂O₂. Natriumpercarbonat liegt als weißes, wasserlösliches Pulver vor, das leicht in Natriumcarbonat und bleichend und oxidierend wirkenden "aktiven" Sauerstoff zerfällt.
Unter Peroxoschwefelsäuren werden Peroxodischwefelsäure und Peroxomonoschwefelsäure (Caro'sche Säure) verstanden.
Vorzugsweise ist das mindestens eine anorganische Salz einer Peroxoschwefelsäure ausgewählt aus Ammoniumperoxodisulfat, Alkalimetallperoxodisulfaten, Ammoniumperoxomonosulfat, Alkalimetallperoxomonosulfaten und Alkalimetallhydrogenperoxomonosulfaten. Besonders bevorzugt sind Ammoniumperoxodisulfat, Kaliumperoxodisulfat, Natriumperoxodisulfat und Kaliumhydrogenperoxomonosulfat. Weiterhin hat es sich bei den Arbeiten zur vorliegenden Erfindung als besonders bevorzugt erwiesen, wenn das erfindungsgemäße Blondierpulver mindestens zwei verschiedene Peroxodisulfate enthält. Bevorzugte Peroxodisulfatsalze sind dabei Kombinationen aus Ammoniumperoxodisulfat und Kaliumperoxodisulfat und/oder Natriumperoxodisulfat.
Bevorzugte erfindungsgemäße Blondierpulver enthalten mindestens ein Oxidationsmittel, das aus Natriumpercarbonaten und anorganischen Salzen einer Peroxoschwefelsäure sowie Mischungen hiervon ausgewählt ist, in einer Gesamtmenge von 5 - 85 Gew.-%, bevorzugt 10 - 75 Gew.-%, besonders bevorzugt 15 - 65 Gew.%, außerordentlich bevorzugt 20 - 55 Gew.-%, jeweils bezogen auf das Gewicht des Blondierpulvers.
Das erfindungsgemäße Blondierpulver enthält als zweiten wesentlichen Bestandteil weiterhin mindestens eine Dicarbonsäure mit 2 bis 10 Kohlenstoffatomen, ausgewählt aus Bernsteinsäure und/oder mindestens einem Salz dieser Säure sowie Mischungen dieser Verbindungen. Erfindungsgemäß geeignete Salze der Dicarbonsäure mit 2 bis 10 Kohlenstoffatomen sind ausgewählt aus den Mono- und Disalzen der Anionen von Bernsteinsäure mit Ammoniumionen, Alkalimetallionen, Erdalkalimetallionen und den Ionen von basischen Aminosäuren, wie Arginin, Lysin und Histidin, insbesondere mit Lithium-, Natrium-, Kalium-, Magnesium- und Calcium-Ionen, sowie Mischungen dieser Salze.
Die erfindungsgemäß besonders bevorzugte Bernsteinsäure weist bei 1013 mbar einen Schmelzpunkt im Bereich von 185 - 187 °C auf, ist also bei 20°C ein Feststoff. Erfindungsgemäß geeignete Salze der Bernsteinsäure sind ausgewählt aus den Succinaten und Hydrogensuccinaten von Ammoniumionen, Alkalimetallionen, Erdalkalimetallionen und den Ionen von basischen Aminosäuren, wie Arginin, Lysin und Histidin, insbesondere den Lithium-, Natrium-, Kalium-, Magnesium- und Calcium-Ionen, oder den Succinaten und Hydrogensuccinaten von basischen Aminosäuren, wie Arginin, Lysin und/oder Histidin, z.B. Argininsuccinat, sowie Mischungen dieser Salze. Die genannten Salze der Bernsteinsäure können auch gebundenes Kristallwasser enthalten, insbesondere das Natriumsuccinathexahydrat, das erfindungsgemäß besonders bevorzugt ist.

Erfindungsgemäß bevorzugte Blondierpulver enthalten Bernsteinsäure und/oder mindestens ein Salz dieser Säure in einer auf die Masse an freier Dicarbonsäure umgerechneten Gesamtmenge von 0,03 - 7 Gew.-%, bevorzugt 0,1 - 5 Gew.-%, besonders bevorzugt 0,5 - 3 Gew.-%, außerordentlich bevorzugt 0,9 - 1,5 Gew.-%, jeweils bezogen auf das Gewicht des Blondierpulvers, wobei Dicarbonsäuren mit 2 bis 10 Kohlenstoffatomen, ausgewählt aus Bernsteinsäure, Äpfelsäure, Oxalsäure, Malonsäure, Adipinsäure, Pimelinsäure, Korksäure, Azelainsäure, Sebacinsäure, Maleinsäure, Fumarsäure, D-Weinsäure, L-Weinsäure, meso-Weinsäure, Traubensäure, alpha-Ketoglutarsäure, beta-Ketoglutarsäure, Oxalessigsäure, und/oder den Salzen dieser Säuren, in einer auf die Masse an freier Dicarbonsäure umgerechneten Gesamtmenge von 0,03 - 7 Gew.-%, bevorzugt 0,1 - 5 Gew.-%, besonders bevorzugt 0,5 - 3 Gew.-%, außerordentlich bevorzugt 0,9 - 1,5 Gew.-%, jeweils bezogen auf das Gewicht des Blondierpulvers, enthalten sind.
Das erfindungsgemäße Blondierpulver enthält als dritten wesentlichen Bestandteil weiterhin Arginin und Lysin oder mindestens eines der Salze dieser Aminosäuren. Unter den erfindungsgemäß bevorzugt geeigneten Salzen von Arginin und Lysin sind die Ammoniumsalze, Alkalimetallsalze und Erdalkalimetallsalze, insbesondere die Lithium-, Natrium-, Kalium-, Magnesium- und Calciumsalze, außerdem die Hydrohalogenide, insbesondere die Hydrochloride, außerdem die Salze mit mindestens einer vorstehend genannten Dicarbonsäure mit 2 bis 10 Kohlenstoffatomen, sowie Mischungen dieser Salze. Ein erfindungsgemäß besonders bevorzugtes Aminosäuresalz ist Lysinhydrochlorid. Die erfindungsgemäß geeigneten Aminosäuren Arginin und Lysin und deren Salze können auch Kristallwasser enthalten.
Erfindungsgemäß bevorzugte Blondierpulver enthalten Arginin und Lysin oder mindestens ein Salz dieser Aminosäuren in einer auf die Masse an freier Aminosäure umgerechneten Gesamtmenge von 0,1 - 7 Gew.-%, bevorzugt 0,2 - 5 Gew.-%, besonders bevorzugt 0,5 - 2,5 Gew.-%, außerordentlich bevorzugt 1 - 2 Gew.-%, jeweils bezogen auf das Gewicht des Blondierpulvers, wobei die Aminosäuren Arginin und Lysin, ggf. Histidin, oder mindestens ein Salz dieser Aminosäuren in einer auf die Masse an freier Aminosäure umgerechneten Gesamtmenge von 0,1 - 7 Gew.-%, bevorzugt 0,2 - 5 Gew.-%, besonders bevorzugt 0,5 - 2,5 Gew.-%, außerordentlich bevorzugt 1 - 2 Gew.-%, jeweils bezogen auf das Gewicht des Blondierpulvers, enthalten sind.
Als erfindungsgemäß außerordentlich bevorzugt hat sich die Kombination aus Bernsteinsäure, Lysin und Arginin erwiesen.

Erfindungsgemäß besonders bevorzugte Blondierpulver enthalten Bernsteinsäure und/oder mindestens ein Bernsteinsäuresalz in einer auf die Masse an freier Dicarbonsäure umgerechneten Gesamtmenge von 0,03 - 7 Gew.-%, bevorzugt 0,1 - 5 Gew.-%, besonders bevorzugt 0,5 - 3 Gew.-%, außerordentlich bevorzugt 0,9 - 1,5 Gew.-%, und weiterhin Arginin und Lysin oder mindestens ein Salz dieser Aminosäuren in einer auf die Masse an freier Aminosäure umgerechneten Gesamtmenge von 0,1 - 7 Gew.-%, bevorzugt 0,2 - 5 Gew.-%, besonders bevorzugt 0,5 - 2,5 Gew.-%, außerordentlich bevorzugt 1 - 2 Gew.-%, wobei sich die Mengenangaben auf das Gewicht des Blondierpulvers beziehen.
Die erfindungsgemäßen Blondierpulver weisen einen Wassergehalt von 0 bis 8 Gew.-%, bevorzugt 0,1 bis 4,5 Gew.-%, besonders bevorzugt 0,5 bis 2,5 Gew.-%, Wasser auf, jeweils bezogen auf das Gewicht des Blondierpulvers. Diese Angaben beziehen sich auf den Gehalt an freiem Wasser. Nicht berücksichtigt ist der Gehalt an molekular gebundenem Wasser oder Kristallwasser, den einzelne Pulverbestandteile aufweisen können.
Der Wassergehalt kann beispielsweise in Anlehnung an ISO 4317 (Version 2011-12) mittels Karl-Fischer-Titration bestimmt werden.
Erfindungsgemäß bevorzugte Blondierpulver enthalten zusätzlich mindestens ein anorganisches, bei 20 °C und 1013 mbar festes Alkalisierungsmittel, das bevorzugt in einer Gesamtmenge von 1 - 60 Gew.-%, bevorzugt 5 - 55 Gew.-%, besonders bevorzugt 10 - 50 Gew.%, außerordentlich bevorzugt 15 - 45 Gew.-%, jeweils bezogen auf das Gewicht des Blondierpulvers, enthalten ist. Erfindungsgemäß besonders bevorzugte anorganische, bei 20 °C und 1013 mbar feste Alkalisierungsmittel sind ausgewählt aus Alkalimetallsilikaten, Erdalkalimetallsilikaten, Erdalkalimetallhydroxidcarbonaten, Erdalkalimetallcarbonaten, Alkalimetallmetasilikaten, Erdalkalimetallmetasilikaten, Alkalimetallhydroxiden, Erdalkalimetallhydroxiden, (Erd-)Alkalimetallphosphaten und (Erd-)Alkalimetallhydrogenphosphaten sowie Mischungen dieser Substanzen. Erfindungsgemäß besonders bevorzugte anorganische, bei 20 °C und 1013 mbar feste Alkalisierungsmittel sind ausgewählt aus Natriummetasilikaten mit einem molaren SiO₂/Na₂O-Verhältnis von ≥ 2, bevorzugt von 2,5 bis 3,5, und aus Magnesiumhydroxidcarbonaten sowie Mischungen dieser Substanzen. Erfindungsgemäß bevorzugte Magnesiumhydroxidcarbonate sind solche mit der Formel MgCO₃ . Mg(OH)₂ · 2 H₂O und solche mit der Formel MgCO₃ · Mg(OH)₂. Magnesiumhydroxidcarbonat mit der Formel MgCO₃ · Mg(OH)₂ ist erfindungsgemäß besonders bevorzugt.
Erfindungsgemäß besonders bevorzugte Blondierpulver enthalten, jeweils bezogen auf ihr Gesamtgewicht, 25 - 50 Gew.-%, bevorzugt 30 - 45 Gew.-%, besonders bevorzugt 34 - 40 Gew.-% Natriumsilikate mit einem molaren SiO₂/Na₂O-Verhältnis von ≥ 2, bevorzugt von 2,5 bis 3,5, und 2 - 20 Gew.-%, bevorzugt 5 - 15 Gew.-%, besonders bevorzugt 8 - 25 Gew.-% Magnesiumhydroxidcarbonat als anorganische, bei 20 °C und 1013 mbar feste Alkalisierungsmittel.
Erfindungsgemäß außerordentlich bevorzugte Blondierpulver enthalten, jeweils bezogen auf ihr Gesamtgewicht, 25 - 50 Gew.-%, bevorzugt 30 - 45 Gew.-%, besonders bevorzugt 34 - 40 Gew.-% Natriumsilikate mit einem molaren SiO₂/Na₂O-Verhältnis von ≥ 2, bevorzugt von 2,5 bis 3,5, und 2 - 20 Gew.-%, bevorzugt 5 - 15 Gew.-%, besonders bevorzugt 10 - 13 Gew.-% Magnesiumhydroxidcarbonat mit der Formel MgCO₃ · Mg(OH)₂ als anorganische, bei 20 °C und 1013 mbar feste Alkalisierungsmittel.

Sofern das erfindungsgemäße oder erfindungsgemäß bevorzugte Blondierpulver ein oder mehrere anorganische Carbonate enthält, sei es als Alkalisierungsmittel oder als Oxidationsmittel in Form von Natriumcarbonat-Wasserstoffperoxid-Komplexen, so ist deren Gehalt bevorzugt so gewählt, dass in der Anwendungsmischung mit der nachstehend diskutierten Oxidationszusammensetzung (Ox) die molare CO₃²-Gesamtkonzentration mindestens 0,015 mol/100 Gramm Anwendungsmischung beträgt.
Sofern das erfindungsgemäße oder erfindungsgemäß bevorzugte Blondierpulver ein oder mehrere anorganische Carbonate enthält, sei es als Alkalisierungsmittel oder als Oxidationsmittel in Form von Natriumcarbonat-Wasserstoffperoxid-Komplexen, so ist deren Gehalt besonders bevorzugt so gewählt, dass in der Anwendungsmischung mit der nachstehend diskutierten Oxidationszusammensetzung (Ox) die molare CO₃²-Gesamtkonzentration rechnerisch mindestens viermal höher ist als die Gesamtkonzentration an Protonendonatoren.
Sofern das erfindungsgemäße oder erfindungsgemäß bevorzugte Blondierpulver ein oder mehrere anorganische Carbonate enthält, sei es als Alkalisierungsmittel oder als Oxidationsmittel in Form von Natriumcarbonat-Wasserstoffperoxid-Komplexen, so ist deren Gehalt außerordentlich bevorzugt so gewählt, dass in der Anwendungsmischung mit der nachstehend diskutierten Oxidationszusammensetzung (Ox) die molare CO₃²-Gesamtkonzentration mindestens 0,015 mol/100 Gramm Anwendungsmischung beträgt und rechnerisch mindestens viermal höher ist als die Gesamtkonzentration an Protonendonatoren.

Zur Entstaubung der erfindungsgemäßen Blondierpulver kann mindestens ein Entstaubungsmittel zugesetzt werden, das insbesondere ausgewählt ist aus mindestens einem Öl, insbesondere ausgewählt aus Paraffinöl, Siliconöl oder Esteröl sowie Mischungen dieser Öle.
Erfindungsgemäß bevorzugte Blondierpulver enthalten daher zusätzlich mindestens ein Öl in einer Gesamtmenge von 0,1 - 15 Gew.-%, bevorzugt 0,5 - 10 Gew.-%, besonders bevorzugt 1 - 8 Gew.-%, außerordentlich bevorzugt 2 - 6 Gew.-%, jeweils bezogen auf das Gewicht des Blondierpulvers. Erfindungsgemäß bevorzugte Öle sind ausgewählt aus natürlichen und synthetischen Kohlenwasserstoffen, besonders bevorzugt aus Paraffinölen, C₁₈-C₃₀-Isoparaffinen, insbesondere Isoeicosan, Polyisobutene und Polydecene, weiterhin ausgewählt aus C₈-C₁₆-Isoparaffinen, insbesondere aus Isodecan, Isododecan, Isotetradecan und Isohexadecan sowie Mischungen hiervon, sowie 1,3-Di-(2-ethylhexyl)-cyclohexan.
Weitere erfindungsgemäß bevorzugte Öle sind ausgewählt aus den Benzoesäureestern von linearen oder verzweigten C8-22-Alkanolen. Besonders bevorzugt sind Benzoesäure-C12-C15-alkylester. Weitere erfindungsgemäß bevorzugte Öle sind ausgewählt aus Fettalkoholen mit 6 - 30 Kohlenstoffatomen, die ungesättigt oder verzweigt und gesättigt oder verzweigt und ungesättigt sind. Bevorzugte Alkoholöle sind 2-Hexyldecanol, 2-Octyldodecanol, 2-Ethylhexylalkohol und Isostearylalkohol. Weitere erfindungsgemäß bevorzugte kosmetische Öle sind ausgewählt aus den Triglyceriden (= Dreifachestern des Glycerins) von linearen oder verzweigten, gesättigten oder ungesättigten, gegebenenfalls hydroxylierten C8-30-Fettsäuren. Besonders bevorzugt kann die Verwendung natürlicher Öle, z.B. Amaranthsamenöl, Aprikosenkernöl, Arganöl, Avocadoöl, Babassuöl, Baumwollsaatöl, Borretschsamenöl, Camelinaöl, Distelöl, Erdnussöl, Granatapfelkernöl, Grapefruitsamenöl, Hanföl, Haselnussöl, Holundersamenöl, Johannesbeersamenöl, Jojobaöl, Leinöl, Macadamianussöl, Maiskeimöl, Mandelöl, Marulaöl, Nachtkerzenöl, Olivenöl, Palmöl, Palmkernöl, Paranussöl, Pekannussöl, Pfirsichkernöl Rapsöl, Rizinusöl, Sanddornfruchtfleischöl, Sanddornkernöl, Sesamöl, Sojaöl, Sonnenblumenöl, Traubenkernöl, Walnussöl, Wildrosenöl, Weizenkeimöl, und die flüssigen Anteile des Kokosöls und dergleichen sein. Bevorzugt sind aber auch synthetische Triglyceridöle, insbesondere Capric/Caprylic Triglycerides.
Weitere erfindungsgemäß besonders bevorzugte kosmetische Öle sind ausgewählt aus den Dicarbonsäureestern von linearen oder verzweigten C₂-C₁₀-Alkanolen, insbesondere Diisopropyladipat, Di-n-butyladipat, Di-(2-ethylhexyl)adipat, Dioctyladipat, Diethyl-/Di-n-butyl/ Dioctylsebacat, Diisopropylsebacat, Dioctylmalat, Dioctylmaleat, Dicaprylylmaleat, Diisooctylsuccinat, Di-2-ethylhexylsuccinat und Di-(2-hexyldecyl)-succinat.
Weitere erfindungsgemäß besonders bevorzugte kosmetische Öle sind ausgewählt aus den Estern der linearen oder verzweigten gesättigten oder ungesättigten Fettalkohole mit 2 - 30 Kohlenstoffatomen mit linearen oder verzweigten gesättigten oder ungesättigten Fettsäuren mit 2 - 30 Kohlenstoffatomen, die hydroxyliert sein können. Dazu zählen bevorzugt 2-Hexyldecylstearat, 2-Hexyldecyllaurat, Isodecylneopentanoat, Isononylisononanoat, 2-Ethylhexylpalmitat und 2-Ethylhexylstearat, Isopropylmyristat, Isopropylpalmitat, Isopropylstearat, Isopropylisostearat, Isopropyloleat, Isooctylstearat, Isononylstearat, Isocetylstearat, Isononylisononanoat, Isotridecylisononanoat, Cetearylisononanoat, 2-Ethylhexyllaurat, 2-Ethylhexylisostearat, 2-Ethylhexylcocoat, 2-Octyldodecylpalmitat, Butyloctansäure-2-butyloctanoat, Diisotridecylacetat, n-Butylstearat, n-Hexyllaurat, n-Decyloleat, Oleyloleat, Oleylerucat, Erucyloleat, Erucylerucat, Ethylenglycoldioleat und Ethylenglycoldipalmitat. Weitere erfindungsgemäß bevorzugte kosmetische Öle sind ausgewählt aus den Anlagerungsprodukten von 1 bis 5 Propylenoxid-Einheiten an ein- oder mehrwertige C₈₋₂₂-Alkanole, wie Octanol, Decanol, Decandiol, Laurylalkohol, Myristylalkohol und Stearylalkohol, z. B. PPG-2-Myristylether und PPG-3-Myristylether. Weitere erfindungsgemäß bevorzugte kosmetische Öle sind ausgewählt aus den Anlagerungsprodukten von mindestens 6 Ethylenoxid- und/oder Propylenoxid-Einheiten an ein- oder mehrwertige C₃₋₂₂-Alkanole wie Glycerin, Butanol, Butandiol, Myristylalkohol und Stearylalkohol, die gewünschtenfalls verestert sein können, z. B. PPG-14-Butylether, PPG-9-Butylether, PPG-10-Butandiol, PPG-15-Stearylether und Glycereth-7-diisononanoat.
Weitere erfindungsgemäß bevorzugte kosmetische Öle sind ausgewählt aus den C₈-C₂₂-Fettalkoholestern einwertiger oder mehrwertiger C₂-C₇-Hydroxycarbonsäuren, insbesondere die Ester der Glycolsäure, Milchsäure, Äpfelsäure, Weinsäure, Citronensäure und Salicylsäure, z. B. C₁₂-C₁₅-Alkyllactat.
Weitere erfindungsgemäß bevorzugte kosmetische Öle sind ausgewählt aus den symmetrischen, unsymmetrischen oder cyclischen Estern der Kohlensäure mit C₃₋₂₂-Alkanolen, C₃₋₂₂-Alkandiolen oder C₃₋₂₂-Alkantriolen, z. B. Dicaprylylcarbonat, oder die Ester gemäß DE 19756454 A1, insbesondere Glycerincarbonat.
Weitere kosmetische Öle, die erfindungsgemäß geeignet sind, sind ausgewählt aus den Siliconölen, zu denen z. B. Dialkyl- und Alkylarylsiloxane, wie beispielsweise Decamethylcyclopentasiloxan, Dodecamethylcyclohexasiloxan, Dimethylpolysiloxan und Methylphenylpolysiloxan, aber auch Hexamethyldisiloxan, Octamethyltrisiloxan und Decamethyltetrasiloxan zählen.
Es kann erfindungsgemäß außerordentlich bevorzugt sein, Mischungen der vorgenannten Öle einzusetzen.
Bevorzugte erfindungsgemäße Blondierpulver sind dadurch gekennzeichnet, dass das kosmetische Öl ausgewählt ist aus natürlichen und synthetischen Kohlenwasserstoffen, besonders bevorzugt aus Paraffinölen, C₁₈-C₃₀-Isoparaffinen, insbesondere Isoeicosan, Polyisobutene und Polydecene, C₈-C₁₆-Isoparaffinen, sowie 1,3-Di-(2-ethylhexyl)-cyclohexan; den Benzoesäureestern von linearen oder verzweigten C₈₋₂₂-Alkanolen; Fettalkoholen mit 6 - 30 Kohlenstoffatomen, die ungesättigt oder verzweigt und gesättigt oder verzweigt und ungesättigt sind; Triglyceriden von linearen oder verzweigten, gesättigten oder ungesättigten, gegebenenfalls hydroxylierten C₈₋₃₀-Fettsäuren, insbesondere natürlichen Ölen; den Dicarbonsäureestern von linearen oder verzweigten C₂-C₁₀-Alkanolen; den Estern der linearen oder verzweigten gesättigten oder ungesättigten Fettalkohole mit 2 - 30 Kohlenstoffatomen mit linearen oder verzweigten gesättigten oder ungesättigten Fettsäuren mit 2 - 30 Kohlenstoffatomen, die hydroxyliert sein können; den Anlagerungsprodukten von 1 bis 5 Propylenoxid-Einheiten an ein- oder mehrwertige C₈₋₂₂-Alkanole; den Anlagerungsprodukten von mindestens 6 Ethylenoxid- und/oder Propylenoxid-Einheiten an ein- oder mehrwertige C₃₋₂₂-Alkanole; den C₈-C₂₂-Fettalkoholestern einwertiger oder mehrwertiger C2-C7-Hydroxycarbonsäuren; den symmetrischen, unsymmetrischen oder cyclischen Estern der Kohlensäure mit C₃₋₂₂-Alkanolen, C₃₋₂₂-Alkandiolen oder C₃₋₂₂-Alkantriolen; den Estern von Dimeren ungesättigter C₁₂-C₂₂-Fettsäuren (Dimerfettsäuren) mit einwertigen linearen, verzweigten oder cyclischen C₂-C₁₈-Alkanolen oder mit mehrwertigen linearen oder verzweigten C₂-C₆-Alkanolen; Siliconölen sowie Mischungen der vorgenannten Substanzen und bevorzugt in einer Gesamtmenge von 0,1 - 15 Gew.-%, bevorzugt 0,5 - 10 Gew.-%, besonders bevorzugt 1 - 8 Gew.-%, außerordentlich bevorzugt 2 - 6 Gew.-%, jeweils bezogen auf das Gewicht des Blondierpulvers, enthalten ist.

Weitere erfindungsgemäß bevorzugte Blondierpulver enthalten mindestens einen Komplexbildner, bevorzugt in einer Gesamtmenge von 0,1 - 3 Gew.-%, bevorzugt 0,2 - 2,5 Gew.-%, besonders bevorzugt 0,5 - 2 Gew.-%, außerordentlich bevorzugt 1 - 1,7 Gew.-%, jeweils bezogen auf das Gewicht des Blondierpulvers, wobei der mindestens eine Komplexbildner besonders bevorzugt ausgewählt ist aus den nachfolgend genannten Säuren und/oder ihren Alkalimetallsalzen: Ethylendiamintetraessigsäure (EDTA); N-Hydroxyethylethylendiamintriessigsäure; Aminotrimethylenphosphonsäure; Diethylentriaminpentaessigsäure; Lauroylethylendiamintriessigsäure; Nitrilotriessigsäure; Iminodibernsteinsäure; N-2-Hydroxyethyliminodiessigsäure; Ethylenglycol-bis-(beta-amino-ethylether)-N,N-tetraessigsäure; Aminotrimethylenphosphonsäure, Pentanatriumaminotrimethylenphosphonat, sowie Mischungen hiervon.

Weitere erfindungsgemäß bevorzugte Blondierpulver enthalten mindestens ein Polymer, das ausgewählt ist aus Acrylsäurehomo- und -copolymeren, Methacrylsäurehomo- und -copolymeren, Itaconsäurehomo- und -copolymeren, Polysacchariden, die chemisch und/oder physikalisch modifiziert sein können, und Mischungen dieser Polymere, wobei besonders bevorzugt eines oder mehrere der genannten Polymere in einer Gesamtmenge von 0,1 - 6 Gew.-%, bevorzugt 0,5 - 4 Gew.-%, besonders bevorzugt 1 - 3,5 Gew.-%, außerordentlich bevorzugt 2 - 3 Gew.-%, jeweils bezogen auf das Gewicht des Blondierpulvers, enthalten ist.

Ein weiterer Gegenstand der vorliegenden Erfindung ist ein Verfahren zur Aufhellung von keratinischen Fasern, insbesondere menschlichen Haaren, bei dem ein erfindungsgemäßes oder erfindungsgemäß bevorzugtes Blondierpulver mit einer Oxidationszusammensetzung vermischt wird, die, jeweils bezogen auf ihr Gewicht, 50 - 96 Gew.-%, bevorzugt 70 - 93 Gew.-%, besonders bevorzugt 80 - 90 Gew.-%, Wasser und 0,5 - 20 Gew.-% Wasserstoffperoxid enthält und weiterhin mindestens ein pH-Stellmittel in einer solchen Menge enthält, dass die Oxidationszusammensetzung einen pH-Wert im Bereich von 2,5 bis 5,5, gemessen bei 20°C, aufweist, unmittelbar danach auf die keratinhaltigen Fasern aufgebracht, 5 bis 60 Minuten auf den Fasern belassen und anschließend die Fasern mit Wasser gespült und optional mit einem tensidhaltigen Reinigungsmittel ausgewaschen werden, wobei das Blondierpulver (B) und die Oxidationszusammensetzung (Ox) bevorzugt in einem gewichtsbezogenen Verhältnis (B) : (Ox) von 0,2 - 1, besonders bevorzugt 0,3 - 0,8, weiter bevorzugt 0,4 - 0,7, außerordentlich bevorzugt 0,5 - 0,6, miteinander vermischt werden.
Die im erfindungsgemäßen Aufhellverfahren eingesetzte Oxidationszusammensetzung (Ox) enthält im Wesentlichen Wasser und Wasserstoffperoxid. Die Konzentration des Wasserstoffperoxids wird einerseits von den gesetzlichen Vorgaben und andererseits von dem gewünschten Effekt bestimmt. Sie beträgt 0,5 - 20 Gew.-%, bevorzugt 3 - 12 Gew.-%, besonders bevorzugt 6 - 9 Gew.-% Wasserstoffperoxid (berechnet als 100%iges H₂O₂), jeweils bezogen auf das Gewicht der Oxidationszusammensetzung (Ox).

Die Oxidationszusammensetzung (Ox) besitzt zur Stabilisierung des Wasserstoffperoxids bevorzugt einen sauren pH-Wert, insbesondere einen pH-Wert im Bereich von 2,5 bis 5,5, gemessen bei 20°C. Zur Stabilisierung des Wasserstoffperoxids sind weiterhin bevorzugt Komplexbildner, Konservierungsmittel und/oder Puffersubstanzen enthalten.
Erfindungsgemäß bevorzugt ist das Blondierpulver so zusammengesetzt, dass die Mischung mit der vorgenannten Oxidationszusammensetzung (Ox), also das anwendungsbereite Farbveränderungsmittel, insbesondere Blondiermittel, einen alkalischen pH-Wert, bevorzugt einen pH-Wert von 8 bis 11,5, besonders bevorzugt einen pH-Wert von 8,5 bis 11, außerordentlich bevorzugt einen pH-Wert von 9,0 bis 10,5, aufweist, jeweils gemessen bei 20 °C.
Erfindungsgemäß besonders bevorzugt verwendete Oxidationszusammensetzungen (Ox) enthalten weiterhin mindestens ein Öl und/oder mindestens eine Fettkomponente mit einem Schmelzpunkt im Bereich von 23 - 110 °C, bevorzugt in einer Gesamtmenge von 0,1 - 60 Gew.-%, besonders bevorzugt 0,5 - 40 Gew.-%, außerordentlich bevorzugt 2 - 24 Gew.-%, jeweils bezogen auf das Gewicht der erfindungsgemäß bevorzugt verwendeten Oxidationszusammensetzung (Ox). Die für die erfindungsgemäß bevorzugt verwendeten Oxidationszusammensetzungen (Ox) geeigneten Öle sind die gleichen Öle, die vorstehend als geeignete Entstaubungsmittel offenbart sind.
In den Oxidationszusammensetzungen (Ox) erfindungsgemäß bevorzugt verwendete Fettkomponenten mit einem Schmelzpunkt im Bereich von 23 - 110 °C sind ausgewählt aus linearen gesättigten 1-Alkanolen mit 12 - 30 Kohlenstoffatomen, bevorzugt in einer Gesamtmenge von 0,1 - 8 Gew.-%, besonders bevorzugt 3,0 bis 6,0 Gew.-%, jeweils bezogen auf das Gewicht der erfindungsgemäß verwendeten Oxidationszusammensetzung (Ox).
Bevorzugt ist das mindestens eine lineare gesättigte 1-Alkanol mit 12 - 30 Kohlenstoffatomen ausgewählt aus Laurylalkohol, Myristylalkohol, Cetylalkohol, Stearylalkohol, Arachidylalkohol, und Behenylalkohol sowie aus Mischungen dieser 1-Alkanole, besonders bevorzugt aus Cetylalkohol, Stearylalkohol und Cetylalkohol/ Stearylalkohol-Mischungen.
Erfindungsgemäß bevorzugt verwendete Oxidationszusammensetzungen (Ox) enthalten weiterhin, jeweils bezogen auf ihr Gewicht, mindestens ein lineares gesättigtes 1-Alkanol mit 12 - 30 Kohlenstoffatomen in einer Gesamtmenge von 0,1 - 8 Gew.-%, bevorzugt in einer Gesamtmenge von 2 - 6 Gew.-%, wobei mindestens ein 1-Alkanol, ausgewählt aus Cetylalkohol, Stearylalkohol und Cetylalkohol/Stearylalkohol-Mischungen, enthalten ist.
Weitere erfindungsgemäß bevorzugt verwendete Oxidationszusammensetzungen (Ox) enthalten mindestens eine Fettkomponente mit einem Schmelzpunkt im Bereich von 23 - 110 °C, die ausgewählt ist aus Estern aus einem gesättigten, einwertigen C₁₆-C₆₀-Alkanol und einer gesättigten C₈-C₃₆-Monocarbonsäure, insbesondere Cetylbehenat, Stearylbehenat und C₂₀-C₄₀-Alkylstearat, Glycerintriestern von gesättigten linearen C₁₂ - C₃₀-Carbonsäuren, die hydroxyliert sein können, Candelillawachs, Carnaubawachs, Bienenwachs, gesättigten linearen C₁₄ - C₃₆-Carbonsäuren sowie Mischungen der vorgenannten Substanzen.

Weitere erfindungsgemäß bevorzugt verwendete Oxidationszusammensetzungen (Ox) enthalten mindestens ein Tensid oder mindestens einen Emulgator, bevorzugt in einer Gesamtmenge von 0,5 - 10 Gew.-%, bevorzugt 1 - 5 Gew.-%, jeweils bezogen auf das Gewicht der erfindungsgemäß verwendeten Oxidationszusammensetzung (Ox).
Tenside und Emulgatoren im Sinne der vorliegenden Anmeldung sind amphiphile (bifunktionelle) Verbindungen, die aus mindestens einem hydrophoben und mindestens einem hydrophilen Molekülteil bestehen. Der hydrophobe Rest ist bevorzugt eine Kohlenwasserstoffkette mit 8-28 KohlenstoffAtomen, die gesättigt oder ungesättigt, linear oder verzweigt sein kann. Besonders bevorzugt ist diese C₈-C₂₈-Alkylkette linear. Basiseigenschaften der Tenside und Emulgatoren sind die orientierte Absorption an Grenzflächen sowie die Aggregation zu Mizellen und die Ausbildung von lyotropen Phasen.
Erfindungsgemäß sind anionische, nichtionische und kationische Tenside besonders geeignet. Aber auch zwitterionische und amphotere Tenside sind erfindungsgemäß sehr geeignet.
Als anionische Tenside eignen sich in den erfindungsgemäßen Zusammensetzungen alle für die Verwendung am menschlichen Körper geeigneten anionischen oberflächenaktiven Stoffe. Diese sind gekennzeichnet durch eine wasserlöslich machende, anionische Gruppe wie beispielsweise eine Carboxylat-, Sulfat-, Sulfonat- oder Phosphat-Gruppe und eine lipophile Alkylgruppe mit 8 bis 30 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind lineare und verzweigte Fettsäuren mit 8 bis 30 C-Atomen (Seifen), Alkylethercarbonsäuren, Acylsarcoside, Acyltauride, Acylisethionate, Sulfobernsteinsäuremono-, -dialkylester und Sulfobernsteinsäuremono-alkylpolyoxyethylester, lineare Alkansulfonate, lineare alpha-Olefinsulfonate, Alkylsulfate und Alkylethersulfate sowie Alkyl- und/oder Alkenylphosphate. Bevorzugte anionische Tenside sind Alkylsulfate, Alkylethersulfate und Alkylethercarbonsäuren mit jeweils 10 bis 18 C-Atomen, bevorzugt 12 bis 14 C-Atomen in der Alkylgruppe und bis zu 12 Glykolethergruppen, bevorzugt 2 bis 6 Glykolethergruppen im Molekül. Beispiele solcher Tenside sind die Verbindungen mit den INCI-Bezeichnungen Sodium Laureth Sulfate, Sodium Lauryl Sulfate, Sodium Myreth Sulfate oder Sodium Laureth Carboxylate.
Als zwitterionische Tenside werden solche oberflächenaktiven Verbindungen bezeichnet, die im Molekül mindestens eine quartäre Ammoniumgruppe und mindestens eine Carboxylat-, Sulfonat- oder Sulfat-Gruppe tragen. Besonders geeignete zwitterionische Tenside sind die so genannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Unter amphoteren Tensiden werden solche oberflächenaktiven Verbindungen verstanden, die außer einer C₈-C₂₄-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete amphotere Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils 8 bis 24 C-Atomen in der Alkylgruppe. Besonders bevorzugte amphotere Tenside sind das N-Kokosalkylaminopropionat, das Kokosacylaminoethylaminopropionat und das C₁₂-C₁₈-Acylsarcosin. Nichtionische Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise Anlagerungsprodukte von 4 bis 50 Mol Ethylenoxid und/oder 0 bis 5 Mol Propylenoxid an lineare und verzweigte Fettalkohole, an Fettsäuren und an Alkylphenole, jeweils mit 8 bis 20 C-Atomen in der Alkylgruppe, ethoxylierte Mono-, Di- und Triglyceride, wie beispielsweise Glycerinmonolaurat + 20 Ethylenoxid, und Glycerinmonostearat + 20 Ethylenoxid, Sorbitanfettsäureester und Anlagerungeprodukte von Ethylenoxid an Sorbitanfettsäureester wie beispielsweise die Polysorbate (Tween 20, Tween 21, Tween 60, Tween 61, Tween 81), Anlagerungsprodukte von Ethylenoxid an Fettsäurealkanolamide und Fettamine, sowie Alkylpolyglycoside. Als nichtionische Tenside eignen sich insbesondere C₈-C₂₂-Alkylmono- und -oligoglycoside und deren ethoxylierte Analoga sowie Ethylenoxid-Anlagerungsprodukte an gesättigte oder ungesättigte lineare Fettalkohole mit jeweils 2 bis 30 Mol Ethylenoxid pro Mol Fettalkohol.
Weitere erfindungsgemäß bevorzugt verwendete Oxidationszusammensetzungen sind dadurch gekennzeichnet, dass das mindestens eine anionische Tensid ausgewählt ist aus Alkylsulfaten, Alkylethersulfaten und Alkylethercarbonsäuren mit jeweils 10 bis 18 C-Atomen, bevorzugt 12 bis 14 C-Atomen in der Alkylgruppe und bis zu 12 Glykolethergruppen, bevorzugt 2 bis 6 Glykolethergruppen, im Molekül.
Weitere erfindungsgemäß bevorzugt verwendete Oxidationszusammensetzungen sind dadurch gekennzeichnet, dass mindestens ein nichtionisches Tensid, ausgewählt aus Ethylenoxid-Anlagerungsprodukten an gesättigte oder ungesättigte lineare Fettalkohole mit jeweils 2 bis 30 Mol Ethylenoxid pro Mol Fettalkohol, sowie mindestens ein anionisches Tensid, ausgewählt aus Alkylsulfaten, Alkylethersulfaten und Alkylethercarbonsäuren mit jeweils 10 bis 18 C-Atomen, bevorzugt 12 bis 14 C-Atomen in der Alkylgruppe und bis zu 12 Glykolethergruppen, bevorzugt 2 bis 6 Glykolethergruppen, im Molekül, enthalten ist, wobei besonders bevorzugt das Gewichtsverhältnis der Gesamtheit aller anionischen Tenside zur Gesamtheit aller nichtionischen Tenside im Bereich von 5 - 50, bevorzugt 10 - 30 liegt.
Als kationische Tenside eignen sich in erfindungsgemäß bevorzugt verwendeten Oxidationszusammensetzungen (Ox) prinzipiell alle für die Verwendung am menschlichen Körper geeigneten kationischen oberflächenaktiven Stoffe. Diese sind gekennzeichnet durch mindestens eine wasserlöslich machende, kationische Gruppe, wie z. B. eine quaternäre Ammonium-Gruppe, oder durch mindestens eine wasserlöslich machende, kationisierbare Gruppe, wie z. B. eine Amin-Gruppe, und weiterhin mindestens eine (lipophil wirkende) Alkylgruppe mit 6 bis 30 C-Atomen oder mindestens eine (lipophil wirkende) Imidazol-Gruppe oder mindestens eine (lipophil wirkende) Imidazylalkyl-Gruppe. Erfindungsgemäß besonders bevorzugt verwendete Oxidationszusammensetzungen (Ox) enthalten mindestens ein kationisches Tensid, das bevorzugt ausgewählt ist aus quarternären Ammoniumverbindungen mit mindestens einem C8-C24-Alkylrest, Esterquats und Amidoaminen mit jeweils mindestens einem C8-C24-Acylrest sowie Mischungen hiervon. Bevorzugte quaternäre Ammoniumverbindungen mit mindestens einem C8-C24-Alkylrest sind Ammoniumhalogenide, insbesondere Chloride, und Ammoniumalkylsulfate, wie Methosulfate oder Ethosulfate, wie C8-C24-Alkyltrimethylammoniumchloride, C8-C24-Dialkyldimethylammoniumchloride und C8-C24-Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27, Quaternium-83, Quaternium-87 und Quaternium-91 bekannten Imidazolium-Verbindungen. Die Alkylketten der oben genannten Tenside weisen bevorzugt 8 bis 24 Kohlenstoffatome auf.
Bei Esterquats handelt es sich um kationische Tenside, die sowohl mindestens eine Esterfunktion als auch mindestens eine quartäre Ammoniumgruppe als Strukturelement und weiterhin mindestens eine C8-C24-Alkylrest oder C8-C24-Acylrest enthalten. Bevorzugte Esterquats sind quaternierte Estersalze von Fettsäuren mit Triethanolamin, quaternierte Estersalze von Fettsäuren mit Diethanolalkylaminen und quaternierten Estersalzen von Fettsäuren mit 1,2-Dihydroxypropyldialkylaminen. Solche Produkte werden beispielsweise unter den Warenzeichen Stepantex®, Dehyquart® und Armocare® vertrieben. N,N-Bis(2-Palmitoyloxyethyl)dimethylammoniumchlorid, Distearoylethyl Dimonium Methosulfate und Distearoylethyl Hydroxyethylmonium Methosulfate sind bevorzugte Beispiele für solche Esterquats.
Die Alkylamidoamine werden üblicherweise durch Amidierung natürlicher oder synthetischer C8-C24-Fettsäuren und Fettsäureschnitte mit Di-(C1-C3)alkylaminoaminen hergestellt. Eine erfindungsgemäß besonders geeignete Verbindung aus dieser Substanzgruppe ist Stearamidopropyldimethylamin.
Erfindungsgemäß besonders bevorzugt verwendete Oxidationszusammensetzungen (Ox) enthalten mindestens ein kationisches Tensid in einer Gesamtmenge von 0,01 - 5 Gew.-%, bevorzugt 0,1 - 3 Gew.-%, besonders bevorzugt 0,3 - 2 Gew.-%, jeweils bezogen auf das Gewicht der erfindungsgemäß verwendeten Oxidationszusammensetzung (Ox).

Ein weiterer Gegenstand der vorliegenden Erfindung ist eine Mehrkomponentenverpackungseinheit (Kit-of-Parts) zur Aufhellung von keratinischen Fasern, die mindestens zwei getrennt voneinander verpackte Komponenten enthält und die dadurch gekennzeichnet ist, dass
i) die erste Komponente (I) ein erfindungsgemäßes oder ein erfindungsgemäß bevorzugtes Blondierpulver ist,
ii) die zweite Komponente (II) eine Oxidationszusammensetzung ist, die, jeweils bezogen auf ihr Gewicht, 50 - 96 Gew.-%, bevorzugt 70 - 93 Gew.-%, besonders bevorzugt 80 - 90 Gew.-%, Wasser und 0,5 - 20 Gew.-% Wasserstoffperoxid enthält und einen pH-Wert im Bereich von 2,5 bis 5,5, gemessen bei 20°C, aufweist,
   wobei die Komponenten (I) und (II) bevorzugt in einem gewichtsbezogenen Verhältnis (I) : (II) von 0,2 - 1, besonders bevorzugt 0,3 - 0,8, weiter bevorzugt 0,4 - 0,7, außerordentlich bevorzugt 0,5 - 0,6, vorliegen.
Ein weiterer Gegenstand der vorliegenden Erfindung ist eine Mehrkomponentenverpackungseinheit (Kit-of-Parts) zur Farbveränderung von keratinischen Fasern, insbesondere menschlichen Haaren, enthaltend mindestens drei getrennt voneinander verpackte Komponenten, die dadurch gekennzeichnet, dass
i) die erste Komponente (I) ein erfindungsgemäßes oder ein erfindungsgemäß bevorzugtes Blondierpulver,
ii) die zweite Komponente (II) eine Oxidationszusammensetzung ist, die, jeweils bezogen auf ihr Gewicht, 50 - 96 Gew.-%, bevorzugt 70 - 93 Gew.-%, besonders bevorzugt 80 - 90 Gew.-%, Wasser und 0,5 - 20 Gew.-% Wasserstoffperoxid enthält und einen pH-Wert im Bereich von 2,5 bis 5,5, gemessen bei 20°C, aufweist,
iii) die dritte Komponente (III) eine Alkalisierungszusammensetzung (Alk) ist, die Wasser und mindestens ein Alkalisierungsmittel, das ausgewählt ist aus Ammoniak, Alkanolaminen und Mischungen hiervon, enthält und einen pH-Wert im Bereich von 8 - 12, bevorzugt von 9 - 11, besonders bevorzugt von 9,5 - 10,5, aufweist, jeweils gemessen bei 20°C,
wobei das Blondierpulver (B), die Oxidationszusammensetzung (Ox) und die Alkalisierungszusammensetzung (Alk) bevorzugt in einem gewichtsbezogenen Verhältnis (B) : (Ox) : (Alk) von (0,7 - 1,3) : (2 - 3) : (2-3), besonders bevorzugt (0,8-1,2) : (2,3-2,7) : (2,3-2,7), außerordentlich bevorzugt 1:2:2, zueinander vorliegen.
Eine Mehrkomponentenverpackungseinheit umfasst mehrere Einzelkomponenten, die getrennt voneinander konfektioniert sind, sowie eine gemeinsame Verpackung für diese Komponenten, zum Beispiel eine Faltschachtel. Darin werden die Komponenten jeweils getrennt in verschiedenen Containern bereitgestellt. Unter Container wird im Rahmen der vorliegenden Erfindung eine Umhüllung verstanden, die in Form einer gegebenenfalls wieder-verschließbaren Flasche, einer Tube, einer Dose, eines Tütchens, eines Sachets oder einer ähnlichen Umhüllung vorliegt. Dem Umhüllungsmaterial sind erfindungsgemäß keine Grenzen gesetzt. Bevorzugt handelt es sich jedoch dabei um Umhüllungen aus Glas oder Kunststoff.
Darüber hinaus kann die Verpackungseinheit Applikationshilfen, wie Kämme, Bürsten oder Pinsel, persönliche Schutzkleidung, insbesondere Einweg-Handschuhe, sowie eine Gebrauchsanleitung umfassen.
In einer weiteren bevorzugten Ausführungsform der Erfindung kann ein erfindungsgemäßes oder ein erfindungsgemäß bevorzugtes Blondierpulver mit einer Alkalisierungszusammensetzung und mit einer Oxidationszusammensetzung zu einem aufhellenden Farbveränderungsmittel für keratinische Fasern kombiniert werden.
Da bei der Behandlung von keratinischer Fasern, insbesondere Haaren, mit Oxidationsmitteln, insbesondere mit Wasserstoffperoxid, der fasereigene Farbstoff Melanin zu einem gewissen Grad zerstört wird, werden die Fasern/Haare zwangsläufig aufgehellt, ändern also ihre Farbe auch ohne die Gegenwart eines Farbstoffs. Daher umfasst der Begriff "Farbveränderung" im Sinne der vorliegenden Anmeldung sowohl die Aufhellung als auch die Färbung mit einem oder mehreren Farbstoffen. Die erfindungsgemäß verwendete Alkalisierungszusammensetzung (Alk) enthält Wasser und mindestens ein Alkalisierungsmittel, das ausgewählt ist aus Ammoniak, Alkanolaminen und Mischungen hiervon, und weist einen pH-Wert im Bereich von 8 - 12, bevorzugt von 9 - 11, besonders bevorzugt von 9,5 - 10,5, auf, jeweils gemessen bei 20°C. Bevorzugte Alkanolamine sind ausgewählt aus Monoethanolamin, 2-Amino-2-methylpropanol und Triethanolamin sowie Mischungen hiervon, wobei Monoethanolamin besonders bevorzugt ist. Ein außerordentlich bevorzugtes Alkalisierungsmittel ist Ammoniak.
Üblicherweise wird Ammoniak (NH₃) in Form seiner wässrigen Lösung eingesetzt. Wässrige Ammoniak-Lösungen enthalten Ammoniak (NH₃) oft in Konzentrationen von 10 bis 32 Gew.-%. Bevorzugt ist hierbei der Einsatz einer wässrigen Ammoniak-Lösung, die 25 Gew.-% Ammoniak (NH₃) enthält. Neben Ammoniak und Alkanolaminen kann mindestens ein weiteres Alkalisierungsmittel enthalten sein, das ausgewählt ist aus Alkalimetallsilikaten, Erdalkalimetallsilikaten, Erdalkalimetallhydroxidcarbonaten, Erdalkalimetallcarbonaten, Alkalimetallmetasilikaten, Erdalkalimetallmetasilikaten, Alkalimetallhydroxiden und Erdalkalimetallhydroxiden, sowie Mischungen dieser Substanzen.
Bevorzugt sind Ammoniak und/oder Monoethanolamin in den erfindungsgemäß bevorzugt verwendeten Alkalisierungszusammensetzungen in Mengen von 0,01 - 10 Gew.-%, bevorzugt von 0,1 - 7,5 Gew.-%, weiter bevorzugt von 0,5 - 5,5 Gew.-% und besonders bevorzugt von 1,5 - 4,5 Gew.-% - jeweils bezogen auf das Gewicht der Alkalisierungszusammensetzung - enthalten.
Ein weiterer Gegenstand der vorliegenden Erfindung ist ein Verfahren zur Farbveränderung von keratinischen Fasern, insbesondere menschlichen Haaren, bei dem ein erfindungsgemäßes oder erfindungsgemäß bevorzugtes Blondierpulver mit einer Oxidationszusammensetzung (Ox), die, jeweils bezogen auf ihr Gewicht, 50 - 96 Gew.-%, bevorzugt 70 - 93 Gew.-%, besonders bevorzugt 80 - 90 Gew.-%, Wasser und 0,5 - 20 Gew.-% Wasserstoffperoxid und weiterhin mindestens ein pH-Stellmittel in einer solchen Menge enthält, dass die Oxidationszusammensetzung einen pH-Wert im Bereich von 2,5 bis 5,5, gemessen bei 20°C, aufweist,
und zusätzlich mit einer Alkalisierungszusammensetzung (Alk), die Wasser und mindestens ein Alkalisierungsmittel, das ausgewählt ist aus Ammoniak, Alkanolaminen und Mischungen hiervon, enthält und einen pH-Wert im Bereich von 8 - 12, bevorzugt von 9 - 11, besonders bevorzugt von 9,5 - 10,5, aufweist, jeweils gemessen bei 20°C,
vermischt wird,
unmittelbar danach auf die keratinhaltigen Fasern aufgebracht, 5 bis 60 Minuten auf den Fasern belassen und anschließend die Fasern mit Wasser gespült und optional mit einem tensidhaltigen Reinigungsmittel ausgewaschen werden, wobei das Blondierpulver (B), die Oxidationszusammensetzung (Ox) und die Alkalisierungszusammensetzung (Alk) bevorzugt in einem gewichtsbezogenen Verhältnis (B) : (Ox) : (Alk) von (0,7 - 1,3) : (2 - 3) : (2 - 3), besonders bevorzugt (0,8 - 1,2) : (2,3 - 2,7) : (2,3 - 2,7), außerordentlich bevorzugt 1:2:2, miteinander vermischt werden.
Erfindungsgemäß bevorzugt ist das Blondierpulver so zusammengesetzt, dass die Mischung mit der vorgenannten Oxidationszusammensetzung (Ox) und mit der vorgenannten Alkalisierungszusammensetzung (Alk), also das anwendungsbereite Farbveränderungsmittel, insbesondere Blondiermittel, einen alkalischen pH-Wert, bevorzugt einen pH-Wert von 8 bis 11,5, besonders bevorzugt einen pH-Wert von 8,5 bis 11, außerordentlich bevorzugt einen pH-Wert von 9,0 bis 10,5, aufweist, jeweils gemessen bei 20 °C.
Die anwendungsbereiten Mischungen aus einem erfindungsgemäßen oder erfindungsgemäß bevorzugten Blondierpulver mit einer der vorgenannten Oxidationszusammensetzungen (Ox) und ggf. mit einer der vorgenannten Alkalisierungszusammensetzungen (Alk) weisen bevorzugt eine Viskosität im Bereich von 15.000 bis 100.000 mPas, besonders bevorzugt 20.000 bis 85.000m Pas, auf, jeweils gemessen bei 20°C mit einem Brookfield-Viskosimeter Typ DV-II+, Spindel 5 mit einer Geschwindigkeit von 4 Umdrehungen/Minute. Eine Viskosität in diesem Bereich erlaubt, dass das anwendungsbereite Mittel sich einerseits gut auftragen lässt und andererseits über ein solches Fließverhalten verfügt, dass es für das Mittel eine ausreichend lange Einwirkzeit am Wirkort auf den keratinischen Fasern garantiert.
Um die Mischbarkeit der erfindungsgemäß verwendeten Alkalisierungszusammensetzung mit dem erfindungsgemäßen oder erfindungsgemäß bevorzugten Blondierpulver und der erfindungsgemäß verwendeten Oxidationszusammensetzung zu erleichtern sowie die Anwendungseigenschaften der resultierenden Anwendungsmischung zu verbessern, enthält die erfindungsgemäß bevorzugt verwendete Alkalisierungszusammensetzung bevorzugt, jeweils bezogen auf ihr Gewicht, mindestens ein Tensid in einer Gesamtmenge von 0,5 - 10 Gew.-%, bevorzugt 2 - 8 Gew.-%.
Die für die erfindungsgemäß bevorzugt verwendeten Alkalisierungszusammensetzungen (Alk) geeigneten Tenside sind ausgewählt aus den gleichen anionischen, kationischen, nichtionischen, amphoteren und zwitterionischen Tensiden und Emulgatoren, die vorstehend als für die bevorzugt verwendeten Oxidationszusammensetzungen (Ox) geeignete Tenside und Emulgatoren offenbart sind.
Erfindungsgemäß besonders bevorzugt verwendete Alkalisierungszusammensetzungen (Alk) enthalten weiterhin mindestens ein Öl und/oder mindestens eine Fettkomponente mit einem Schmelzpunkt im Bereich von 23 - 110 °C, bevorzugt in einer Gesamtmenge von 0,1 - 60 Gew.-%, besonders bevorzugt 0,5 - 40 Gew.-%, außerordentlich bevorzugt 2 - 24 Gew.-%, jeweils bezogen auf das Gewicht der erfindungsgemäß bevorzugt verwendeten Alkalisierungszusammensetzung (Alk). Die für die erfindungsgemäß bevorzugt verwendeten Alkalisierungszusammensetzungen (Alk) geeigneten Öle sind die gleichen Öle, die vorstehend als geeignete Entstaubungsmittel offenbart sind.
In den Alkalisierungszusammensetzungen (Alk) erfindungsgemäß bevorzugt verwendete Fettkomponenten mit einem Schmelzpunkt im Bereich von 23 - 110 °C sind ausgewählt aus linearen gesättigten 1-Alkanolen mit 12 - 30 Kohlenstoffatomen, bevorzugt in einer Gesamtmenge von 0,1 - 20 Gew.-%, besonders bevorzugt 3 - 15 Gew.-%, außerordentlich bevorzugt 5 - 10 Gew.-%, jeweils bezogen auf das Gewicht der erfindungsgemäß verwendeten Alkalisierungszusammensetzung. Bevorzugt ist das mindestens eine lineare gesättigte 1-Alkanol mit 12 - 30 Kohlenstoffatomen ausgewählt aus Laurylalkohol, Myristylalkohol, Cetylalkohol, Stearylalkohol, Arachidylalkohol, und Behenylalkohol sowie aus Mischungen dieser 1-Alkanole, besonders bevorzugt aus Cetylalkohol, Stearylalkohol und Cetylalkohol/ Stearylalkohol-Mischungen.
Erfindungsgemäß bevorzugt verwendete Alkalisierungszusammensetzungen (Alk) enthalten weiterhin, jeweils bezogen auf ihr Gewicht, mindestens ein lineares gesättigtes 1-Alkanol mit 12 - 30 Kohlenstoffatomen in einer Gesamtmenge von 0,1 - 20 Gew.-%, bevorzugt in einer Gesamtmenge von 3 - 15Gew.-%, außerordentlich bevorzugt 5 - 10 Gew.-%, wobei mindestens ein 1-Alkanol, ausgewählt aus Cetylalkohol, Stearylalkohol und Cetylalkohol/Stearylalkohol-Mischungen, enthalten ist. Weitere erfindungsgemäß bevorzugt verwendete Alkalisierungszusammensetzungen (Alk) enthalten mindestens eine Fettkomponente mit einem Schmelzpunkt im Bereich von 23 - 110 °C, die ausgewählt ist aus Estern aus einem gesättigten, einwertigen C₁₆-C₆₀-Alkanol und einer gesättigten C₈-C₃₆-Monocarbonsäure, insbesondere Cetylbehenat, Stearylbehenat und C₂₀-C₄₀-Alkylstearat, Glycerintriestern von gesättigten linearen C₁₂ - C₃₀-Carbonsäuren, die hydroxyliert sein können, Candelillawachs, Carnaubawachs, Bienenwachs, gesättigten linearen C₁₄ - C₃₆-Carbonsäuren sowie Mischungen der vorgenannten Substanzen.
Weiterhin können die erfindungsgemäßen oder erfindungsgemäß bevorzugten Blondierpulver und/ oder die erfindungsgemäß bevorzugt verwendeten Alkalisierungszusammensetzungen mindestens einen direktziehenden Farbstoff enthalten. Dabei handelt es sich um Farbstoffe, die direkt auf das Haar aufziehen und keinen oxidativen Prozess zur Ausbildung der Farbe benötigen. Zur Mattierung von durch Melaninabbauprodukte hervorgerufene unerwünschte Restfarbeindrücke, insbesondere im rötlichen oder bläulichen Bereich, sind besonders bevorzugt bestimmte direktziehende Farbstoffe der komplementären Farben enthalten. Direktziehende Farbstoffe sind üblicherweise Nitrophenylendiamine, Nitroaminophenole, Azofarbstoffe, Anthrachinone oder Indophenole. Direktziehende Farbstoffe sind als anionische, kationische und nichtionische direktziehende Farbstoffe bekannt. Die direktziehenden Farbstoffe sind jeweils bevorzugt in einer Menge von 0,001 bis 2 Gew.-%, bezogen auf das Gewicht des Blondierpulvers oder der Alkalisierungszusammensetzung (Alk), eingesetzt. Bevorzugte anionische direktziehende Farbstoffe sind die unter den internationalen Bezeichnungen bzw. Handelsnamen Acid Yellow 1, Yellow 10, Acid Yellow 23, Acid Yellow 36, Acid Orange 7, Acid Red 33, Acid Red 52, Pigment Red 57:1, Acid Blue 7, Acid Green 50, Acid Violet 43, Acid Black 1, Acid Black 52, Bromphenolblau und Tetrabromphenolblau bekannten Verbindungen. Bevorzugte kationische direktziehende Farbstoffe sind kationische Triphenylmethanfarbstoffe, beispielsweise Basic Blue 7, Basic Blue 26, Basic Violet 2 und Basic Violet 14, aromatische Systeme, die mit einer quaternären Stickstoffgruppe substituiert sind, wie beispielsweise Basic Yellow 57, Basic Red 76, Basic Blue 99, Basic Brown 16 und Basic Brown 17, kationische Anthrachinonfarbstoffe, wie HC Blue 16 (Bluequat B) sowie direktziehende Farbstoffe, die einen Heterozyklus enthalten, der mindestens ein quaternäres Stickstoffatom aufweist, insbesondere Basic Yellow 87, Basic Orange 31 und Basic Red 51. Die kationischen direktziehenden Farbstoffe, die unter dem Warenzeichen Arianor vertrieben werden, sind erfindungsgemäß ebenfalls bevorzugte kationische direktziehende Farbstoffe. Als nichtionische direktziehende Farbstoffe eignen sich insbesondere nichtionische Nitro- und Chinonfarbstoffe und neutrale Azofarbstoffe. Bevorzugte nichtionische direktziehende Farbstoffe sind die unter den internationalen Bezeichnungen bzw. Handelsnamen HC Yellow 2, HC Yellow 4, HC Yellow 5, HC Yellow 6, HC Yellow 12, HC Orange 1, Disperse Orange 3, HC Red 1, HC Red 3, HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9 bekannten Verbindungen, sowie 1,4-Diamino-2-nitrobenzol, 2-Amino-4-nitrophenol, 1,4-Bis-(2-hydroxyethyl)-amino-2-nitrobenzol, 3-Nitro-4-(2-hydroxyethyl)aminophenol, 2-(2-Hydroxyethyl)amino-4,6-dinitrophenol, 4-[(2-Hydroxyethyl)amino]-3-nitro-1-methylbenzol, 1-Amino-4-(2-hydroxyethyl)amino-5-chlor-2-nitrobenzol, 4-Amino-3-nitrophenol, 1-(2'-Ureidoethyl)amino-4-nitrobenzol, 2-[(4-Amino-2-nitrophenyl)amino]-benzoesäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chlor-6-ethylamino-4-nitrophenol. Erfindungsgemäß ganz besonders bevorzugt ist eine Kombination aus Tetrabromphenolblau und Acid Red 92 enthalten.
Als weiteren optionalen Inhaltsstoff enthält die erfindungsgemäß bevorzugt verwendete Alkalisierungszusammensetzung mindestens ein Oxidationsfarbstoffvorprodukt, das vorzugsweise aus einer oder mehreren Entwicklerkomponenten und gegebenenfalls einer oder mehreren Kupplerkomponenten ausgewählt ist.
Besonders bevorzugt ist mindestens ein Oxidationsfarbstoffvorprodukt in einer Gesamtmenge von 0,0001 bis 10,0 Gew.-%, bevorzugt 0,001 bis 8 Gew.-%, jeweils bezogen auf das Gewicht der erfindungsgemäß bevorzugt verwendeten Alkalisierungszusammensetzung, enthalten.
Es kann erfindungsgemäß bevorzugt sein, als Entwicklerkomponente mindestens eine Verbindung aus der Gruppe auszuwählen, die gebildet wird aus p-Phenylendiamin, p-Toluylendiamin, 2-(2-Hydroxyethyl)-p-phenylendiamin, 2-(1,2-Dihydroxyethyl)-p-phenylendiamin, N,N-Bis-(2-hydroxyethyl)-p-phenylendiamin, N-(4-Amino-3-methylphenyl)-N-[3-(1H-imidazol-1-yl)propyl]amin, N,N'-Bis-(2-hydroxyethyl)-N,N'-bis-(4-aminophenyl)-1,3-diamino-propan-2-ol, Bis-(2-hydroxy-5-aminophenyl)methan, 1,3-Bis-(2,5-diaminophenoxy)-propan-2-ol, N,N'-Bis-(4-aminophenyl)-1,4-diazacycloheptan, 1,10-Bis-(2,5-diaminophenyl)-1,4,7,10-tetraoxadecan, p-Aminophenol, 4-Amino-3-methylphenol, 4-Amino-2-aminomethylphenol, 4-Amino-2-(1,2-dihydroxyethyl)phenol, 4-Amino-2-(diethylaminomethyl)phenol, 4,5-Diamino-1-(2-hydroxyethyl)pyrazol, 2,4,5,6-Tetraaminopyrimidin, 4-Hydroxy-2,5,6-triaminopyrimidin, 2-Hydroxy-4,5,6-triaminopyrimidin sowie deren physiologisch verträglichen Salzen.
Bevorzugt ist mindestens eine Entwicklerkomponente in einer Gesamtmenge von 0,0001 bis 10,0 Gew.-%, bevorzugt 0,001 bis 8 Gew.-%, jeweils bezogen auf das Gewicht der erfindungsgemäß bevorzugt verwendeten Alkalisierungszusammensetzung, enthalten.
Kupplerkomponenten bilden im Rahmen der oxidativen Färbung allein keine signifikante Färbung aus, sondern benötigen stets die Gegenwart von Entwicklerkomponenten. Daher ist es erfindungsgemäß bevorzugt, dass bei Verwendung mindestens einer Entwicklerkomponente zusätzlich mindestens eine Kupplerkomponente zum Einsatz kommt.
Erfindungsgemäß bevorzugte Kupplerkomponenten sind ausgewählt aus 3-Aminophenol, 5-Amino-2-methylphenol, N-Cyclopentyl-3-aminophenol, 3-Amino-2-chlor-6-methylphenol, 2-Hydroxy-4-aminophenoxyethanol, 2,6-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5-(2-Hydroxyethyl)amino-2-methylphenol, 3-(Diethylamino)phenol, N-Cyclopentyl-3-aminophenol, 1,3-Dihydroxy-5-(methylamino)benzol, 3-Ethylamino-4-methylphenol, 2,4-Dichlor-3-aminophenol, 2-(2,4-Diaminophenoxy)ethanol, 1,3-Bis-(2,4-diaminophenoxy)propan, 1-Methoxy-2-amino-4-(2-hydroxyethylamino)benzol, 1,3-Bis-(2,4-diaminophenyl)propan, 2,6-Bis-(2'-hydroxyethylamino)-1-methylbenzol, 2-({3-[(2-Hydroxyethyl)amino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxyethoxy)-5-methylphenylamin, 1-Amino-3-bis-(2-hydroxyethyl)aminobenzol, Resorcin, Resorcinmonomethylether, 2-Methylresorcin, 5-Methylresorcin, 2,5-Dimethylresorcin, 2-Chlorresorcin, 4-Chlorresorcin, Pyrogallol, 1,2,4-Trihydroxybenzol, 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin, 3,5-Diamino-2,6-dimethoxypyridin, 2,6-Dihydroxy-3,4-dimethylpyridin, 3,4-Diaminopyridin, 2-(2-Methoxyethyl)amino-3-amino-6-methoxypyridin, 2-(4'-Methoxyphenyl)amino-3-aminopyridin, 1-Naphthol, 2-Methyl-1-naphthol, 2-Hydroxymethyl-1-naphthol, 2-Hydroxyethyl-1-naphthol, 1,3-Dihydroxynaphthalin, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin, 2,3-Dihydroxynaphthalin, 4-Hydroxyindol, 6-Hydroxyindol, 7-Hydroxyindol, 4-Hydroxyindolin, 6-Hydroxyindolin, 7-Hydroxyindolin, 4,6-Diaminopyrimidin, 4-Amino-2,6-dihydroxypyrimidin, 2,4-Diamino-6-hydroxypyrimidin, 2,4,6-Trihydroxypyrimidin, 2-Amino-4-methylpyrimidin, 2-Amino-4-hydroxy-6-methylpyrimidin und 4,6-Dihydroxy-2-methylpyrimidin oder Gemischen dieser Verbindungen oder deren physiologisch verträglichen Salzen.
Bevorzugt ist mindestens eine Kupplerkomponente in einer Gesamtmenge von 0,0001 bis 10,0 Gew.-%, bevorzugt 0,001 bis 8 Gew.-%, jeweils bezogen auf das Gewicht der erfindungsgemäß bevorzugt verwendeten Alkalisierungszusammensetzung, enthalten.
Dabei werden Entwicklerkomponenten und Kupplerkomponenten im Allgemeinen in etwa äquimolaren Mengen zueinander eingesetzt. Wenn sich auch der äquimolare Einsatz als zweckmäßig erwiesen hat, so ist ein gewisser Überschuss einzelner Oxidationsfarbstoffvorprodukte nicht nachteilig, so dass Entwicklerkomponenten und Kupplerkomponenten in einem Mol-Verhältnis von 0,2 - 2, insbesondere 0,5 - 1, enthalten sein können.
Bevorzugt beträgt die Einwirkzeit 5 bis 60 min, insbesondere 5 bis 50 min, besonders bevorzugt 10 bis 45 min. Während der Einwirkzeit der Mittel auf der Faser kann es vorteilhaft sein, den Aufhell- oder Farbveränderungsvorgang durch Wärmezufuhr zu unterstützen. Eine Einwirkphase bei Raumtemperatur ist ebenfalls erfindungsgemäß. Insbesondere liegt die Temperatur während der Einwirkzeit zwischen 20 °C und 40 °C, insbesondere zwischen 25 °C und 38 °C. Die Mittel ergeben bereits bei physiologisch verträglichen Temperaturen von unter 45°C gute Behandlungsergebnisse.
Nach Ende des Farbveränderungsvorgangs werden alle auf den Keratinfasern befindlichen Komponenten mit Wasser oder einem tensidhaltigen Reinigungsmittel aus dem Haar gespült. Als Reinigungsmittel kann dabei insbesondere handelsübliches Shampoo dienen, wobei insbesondere dann auf das Reinigungsmittel verzichtet werden kann und der Ausspülvorgang mit Leitungswasser erfolgen kann, wenn das Farbveränderungsmittel einen höheren Tensidgehalt aufweist.
Ein weiterer Gegenstand der vorliegenden Erfindung ist die Verwendung einer Kombination aus Bernsteinsäure und/oder mindestens einem Salz dieser Säure sowie Mischungen dieser Verbindungen, in Kombination mit Arginin und Lysin oder mindestens einem der Salze dieser Aminosäuren, in einem Blondierpulver, das mindestens ein Oxidationsmittel, ausgewählt aus Natriumpercarbonaten und anorganischen Salzen einer Peroxoschwefelsäure, und sowie Mischungen hiervon, und weiterhin 0 bis 8 Gew.-% Wasser, bezogen auf das Gewicht des Blondierpulvers, enthält,
zur Verringerung der Schädigung an keratinischen Fasern, insbesondere menschlichen Haaren, die bewirkt wird durch die Behandlung dieser Fasern mit einer Mischung aus dem Blondierpulver und einer Oxidationszusammensetzung, die, jeweils bezogen auf ihr Gewicht, 50 - 96 Gew.-%, bevorzugt 70 - 93 Gew.-%, besonders bevorzugt 80 - 90 Gew.-%, Wasser und 0,5 - 20 Gew.-% Wasserstoffperoxid enthält und einen pH-Wert im Bereich von 2,5 bis 5,5, gemessen bei 20°C, aufweist.
Das zu den erfindungsgemäßen und erfindungsgemäß bevorzugten Blondierpulvern Gesagte gilt mutatis mutandis auch für die erfindungsgemäßen und erfindungsgemäß bevorzugten Mehrkomponentenverpackungseinheiten (Kits of parts).

Das zu den erfindungsgemäßen und erfindungsgemäß bevorzugten Blondierpulvern Gesagte gilt mutatis mutandis auch für die erfindungsgemäßen und erfindungsgemäß bevorzugten Verfahren zur Aufhellung und/oder zur Farbveränderung der keratinischen Fasern.
Das zu den erfindungsgemäß und erfindungsgemäß bevorzugt verwendeten Oxidationszusammensetzungen oder Alkalisierungszusammensetzungen Gesagte gilt mutatis mutandis auch für die erfindungsgemäßen und erfindungsgemäß bevorzugten Mehrkomponentenverpackungseinheiten (Kits of parts).
Das zu den erfindungsgemäß und erfindungsgemäß bevorzugt verwendeten Oxidationszusammensetzungen oder Alkalisierungszusammensetzungen Gesagte gilt mutatis mutandis auch für die erfindungsgemäßen und erfindungsgemäß bevorzugten Verfahren zur Aufhellung und/oder zur Farbveränderung der keratinischen Fasern.
Das zu den erfindungsgemäßen und erfindungsgemäß bevorzugten Blondierpulvern Gesagte gilt mutatis mutandis auch für die erfindungsgemäße Verwendung.
Das zu den erfindungsgemäß und erfindungsgemäß bevorzugt verwendeten Oxidationszusammensetzungen oder Alkalisierungszusammensetzungen Gesagte gilt mutatis mutandis auch für die erfindungsgemäße Verwendung.

### BEISPIELE

### 1.1 Entwickleremulsion

| Inhaltsstoff | Menge (Gew.-%) |
|---|---|
| Dipicolinsäure (2,6-Dicarboxypyridin) | 0,1 |
| Kaliumhydroxid | 0,15 |
| Etidronsäure | 0,2 |
| Natriumcetearylsulfat | 0,4 |
| Cetearylalkohol | 3,5 |
| PEG-40 Castor Oil | 0,8 |
| Paraffinum liquidum | 17,0 |
| Dinatriumpyrophosphat | 0,1 |
| Natriumbenzoat | 0,04 |
| Wasserstoffperoxid | 9,0 |
| Wasser | ad 100 |

### 1.2 Blondierpulver-Formulierungen

### (soweit nicht anders angegeben entsprechen die Mengenangaben Gewichts-%)

| | Nr. 1 (V) | Nr. 2 (V) | Nr. 3 (E) | Nr. 4 (E) |
|---|---|---|---|---|
| Kaliumpersulfat | 32,00 | 32,00 | 32,00 | 32,00 |
| Ammoniumpersulfat | 10,00 | 10,00 | 10,00 | 10,00 |
| Bernsteinsäure | - | - | 1,00 | 2,00 |
| L-Arginin | - | 1,00 | 0,20 | 0,40 |
| Lysinhydrochlorid | - | - | 0,20 | 0,40 |
| Natriumsilikat mit SiO₂/Na₂O (molar) von 2,61-2,70 | 36,00 | 36,00 | 36,00 | 36,00 |
| Magnesiumhydroxidcarbonat | 13,45 | 10,25 | 10,35 | 8,95 |
| Natriumhexametaphosphat | 0,20 | 0,20 | 0,20 | 0,20 |
| Methylmethacrylat/Methacrylsäure-Copolymer | 1,00 | 1,00 | 1,00 | 1,00 |
| Carboxymethylcellulose | 2,00 | 2,00 | 2,00 | 2,00 |
| EDTA Na2 | 0,60 | 0,60 | 0,60 | 0,60 |
| EDTA Na4 | - | 1,60 | 1,60 | 1,60 |
| Polyquaternium-10 | - | 0,50 | - | - |
| hydrophile Kieselsäure | 0,40 | 0,40 | 0,40 | 0,40 |
| CI 77007 (Ultramarines) | 0,15 | 0,15 | 0,15 | 0,15 |
| Paraffinum Liquidum | 3,60 | 4,30 | 4,30 | 4,30 |
| Parfüm | 0,60 | 0,60 | 0,60 | 0,60 |
| Gesamt | 100,00 | 100,00 | 100,00 | 100,00 |
| Differenz Zugfestigkeit (Young-Modul) nach zwei Blondierungen (x 10⁹) | - (1,69 ± 0,22) | - (1,45 ± 0,13) | - (1,22 ± 0,12) | - (1,28 ± 0,12) |

Das jeweilige Blondierpulver und die Entwickleremulsion wurden im Gewichtsverhältnis 1:2 miteinander vermischt.

Alle Blondierungsbehandlungen führen zu einem Verlust der Haarfaser an Zugfestigkeit. Mit den erfindungsgemäßen Zusammensetzungen E3 und E4 kann dieser Zugfestigkeitsverlust allerdings statistisch signifikant verringert werden.

### 2. Anwendung

100 g der frisch hergestellten Mischung aus dem jeweiligen Blondierpulver und die Entwickleremulsion wurden auf trockene Haarsträhnen appliziert (4 g Anwendungsmischung pro Gramm Haar). Nachdem die Strähnen 45 Minuten lang bei 32 °C blondiert wurden, wurden sie 2 Minuten lang mit Wasser ausgewaschen und mit einem Föhn getrocknet.
Dieser Blondiervorgang wurde einmal wiederholt, so dass die Strähnen insgesamt zweimal nacheinander blondiert wurden.

### 3. Messungen der Haarzugfestigkeit

### Hintergrund

Der Young-Modul ist auch bekannt als Elastizitätsmodul (E-Modul). Er entspricht dem Verhältnis von Spannung ("stress") zu Dehnung ("strain") bei linear elastischem Verhalten (in der Hooke'schen Region).

Das Hooke'sche Elastizitätsgesetz besagt, dass die longitudinale Änderung eines Körpers (Dehnung oder "strain") linear abhängig ist von der Kraft, die die Deformation bewirkt (Spannung oder "stress"). Für feuchtes Haar besteht die lineare Korrelation für eine Dehnung von 0 bis 2%.

Der Young-Modul ist ein Maß für die Stärke einer Faser (je höher der Wert für den Young-Modul, desto stärker ist die Faser).

Die für die Messungen verwendeten Strähnen bestehen aus 40 Fasern (Kerling International (Backnang, Germany), European Natural Hair 7/0; Charge #2014, Mischung 138).

Zunächst wird die mittlere Querschnittfläche jedes einzelnen Haars bestimmt (Universal-Dimensions-Measuring-Device UDM 5000A, (Zimmer GmbH, Darmstadt)), und zwar bei einer Temperatur von 22 °C und einer relative Luftfeuchtigkeit von 50%. Diese Werte werden für die Berechnung der Spannungswerte ("stress values") benötigt.

### 3.2. Bestimmung des Young-Moduls vor der Applikation des Blondiermittels

Alle Haarfasern wurden eine Stunde lang in Wasser eingeweicht. Anschließend wurden sie mit dem Stress-Strain-System MTT 680 mit Kontrolleinheit UV 1000 (Dia-Stron Ltd, UK) mit einer konstanten Geschwindigkeit von 10 mm/min innerhalb der elastischen Phase gedehnt (0-1.5% Verlängerung). Anschließend wurde der E-Modul (Young-Modul) berechnet (Software: UvWin 1.32.1000 (Dia-Stron Ltd, UK).

### 3.3. Bestimmung des Young-Moduls nach der Applikation des Blondiermittels

Nach den vier Blondierbehandlungen wurden die Haarfasern mindestens 48 Stunden lang gelagert. Die Haarfasern wurden eine Stunde lang in Wasser eingeweicht. Anschließend wurden sie mit dem Stress-Strain-System MTT 680 mit Kontrolleinheit UV 1000 (Dia-Stron Ltd, UK) mit einer konstanten Geschwindigkeit von 10 mm/min innerhalb der elastischen Phase gedehnt (0-1.5% Verlängerung). Anschließend wurde der E-Modul (Young-Modul) berechnet (Software: UvWin 1.32.1000 (Dia-Stron Ltd, UK).

## Patentansprüche

1. Blondierpulver, enthaltend
a) mindestens ein Oxidationsmittel, ausgewählt aus Natriumpercarbonaten und anorganischen Salzen einer Peroxoschwefelsäure, und sowie Mischungen hiervon,
b) weiterhin mindestens eine Dicarbonsäure mit 2 bis 10 Kohlenstoffatomen, ausgewählt aus Bernsteinsäure und/oder mindestens einem Salz dieser Säure sowie Mischungen dieser Verbindungen,
c) weiterhin Arginin und Lysin oder mindestens eines der Salze dieser Aminosäuren,
d) und 0 bis 8 Gew.-% Wasser, bezogen auf das Gewicht des Blondierpulvers.

2. Blondierpulver gemäß Anspruch 1, **dadurch gekennzeichnet, dass** das mindestens eine Oxidationsmittel a) in einer Gesamtmenge von 5 - 85 Gew.-%, bevorzugt 10 - 75 Gew.-%, besonders bevorzugt 15 - 65 Gew.%, außerordentlich bevorzugt 20 - 55 Gew.-%, jeweils bezogen auf das Gewicht des Blondierpulvers, enthalten ist.

3. Blondierpulver gemäß Anspruch 1 oder 2, **dadurch gekennzeichnet, dass** Bernsteinsäure und/oder mindestens ein Salz dieser Säure in einer auf die Masse an freier Dicarbonsäure umgerechneten Gesamtmenge von 0,03 - 7 Gew.-%, bevorzugt 0,1 - 5 Gew.-%, besonders bevorzugt 0,5 - 3 Gew.-%, außerordentlich bevorzugt 0,9 - 1,5 Gew.-%, jeweils bezogen auf das Gewicht des Blondierpulvers, enthalten ist,
wobei Dicarbonsäuren mit 2 bis 10 Kohlenstoffatomen, ausgewählt aus Bernsteinsäure, Äpfelsäure, Oxalsäure, Malonsäure, Adipinsäure, Pimelinsäure, Korksäure, Azelainsäure, Sebacinsäure, Maleinsäure, Fumarsäure, D-Weinsäure, L-Weinsäure, meso-Weinsäure, Traubensäure, alpha-Ketoglutarsäure, beta-Ketoglutarsäure, Oxalessigsäure, und/oder den Salzen dieser Säuren, in einer auf die Masse an freier Dicarbonsäure umgerechneten Gesamtmenge von 0,03 - 7 Gew.-%, bevorzugt 0,1 - 5 Gew.-%, besonders bevorzugt 0,5 - 3 Gew.-%, außerordentlich bevorzugt 0,9 - 1,5 Gew.-%, jeweils bezogen auf das Gewicht des Blondierpulvers, enthalten sind.

4. Blondierpulver gemäß einem der Ansprüche 1 - 3, **dadurch gekennzeichnet, dass** Arginin und Lysin oder mindestens ein Salz dieser Aminosäuren in einer auf die Masse an freier Aminosäure umgerechneten Gesamtmenge von 0,1 - 7 Gew.-%, bevorzugt 0,2 - 5 Gew.-%, besonders bevorzugt 0,5 - 2,5 Gew.-%, außerordentlich bevorzugt 1 - 2 Gew.-%, jeweils bezogen auf das Gewicht des Blondierpulvers, enthalten sind,
wobei die Aminosäuren Arginin und Lysin, ggf. Histidin, oder mindestens ein Salz dieser Aminosäuren in einer auf die Masse an freier Aminosäure umgerechneten Gesamtmenge von 0,1 - 7 Gew.-%, bevorzugt 0,2 - 5 Gew.-%, besonders bevorzugt 0,5 - 2,5 Gew.-%, außerordentlich bevorzugt 1 - 2 Gew,-%, jeweils bezogen auf das Gewicht des Blondierpulvers, enthalten sind.

5. Blondierpulver gemäß einem der Ansprüche 1 - 4, **dadurch gekennzeichnet, dass** Bernsteinsäure und/oder mindestens ein Bernsteinsäuresalz in einer auf die Masse an freier Dicarbonsäure umgerechneten Gesamtmenge von 0,03 - 7 Gew.-%, bevorzugt 0,1 - 5 Gew.-%, besonders bevorzugt 0,5 - 3 Gew.-%, außerordentlich bevorzugt 0,9 - 1,5 Gew.-%, und weiterhin Arginin und Lysin oder mindestens ein Salz dieser Aminosäuren in einer auf die Masse an freier Aminosäure umgerechneten Gesamtmenge von 0,1 - 7 Gew.-%, bevorzugt 0,2 - 5 Gew.-%, besonders bevorzugt 0,5 - 2,5 Gew.-%, außerordentlich bevorzugt 1 - 2 Gew.-%, enthalten sind, wobei sich die Mengenangaben auf das Gewicht des Blondierpulvers beziehen.

6. Blondierpulver gemäß einem der Ansprüche 1 - 5, **dadurch gekennzeichnet, dass** zusätzlich mindestens ein anorganisches, bei 20 °C und 1013 mbar festes Alkalisierungsmittel enthalten ist, das bevorzugt in einer Gesamtmenge von 1 - 60 Gew.-%, bevorzugt 5 - 55 Gew.-%, besonders bevorzugt 10 - 50 Gew.%, außerordentlich bevorzugt 15 - 45 Gew.-%, jeweils bezogen auf das Gewicht des Blondierpulvers, enthalten ist.

7. Blondierpulver gemäß einem der Ansprüche 1 - 6, **dadurch gekennzeichnet, dass** das mindestens eine Oxidationsmittel a) ausgewählt ist aus Natriumperoxodisulfat, Kaliumperoxodisulfat, Ammoniumperoxodisulfat, Natriumpercarbonat, Natriumperoxomonosulfat, Kaliumperoxomonosulfat, Ammoniumperoxomonosulfat, sowie Mischungen dieser Verbindungen, bevorzugt Mischungen aus Kaliumperoxodisulfat und Ammoniumperoxodisulfat oder Mischungen aus Natriumperoxodisulfat und Ammoniumperoxodisulfat.

8. Blondierpulver gemäß einem der Ansprüche 1 - 7, **dadurch gekennzeichnet, dass** mindestens ein Öl in einer Gesamtmenge von 0,1 - 15 Gew.-%, bevorzugt 0,5 - 10 Gew.-%, besonders bevorzugt 1 - 8 Gew.-%, außerordentlich bevorzugt 2 - 6 Gew.-%, jeweils bezogen auf das Gewicht des Blondierpulvers, enthalten ist.

9. Blondierpulver gemäß einem der Ansprüche 1 - 8, **dadurch gekennzeichnet, dass** mindestens ein Komplexbildner enthalten ist, bevorzugt in einer Gesamtmenge von 0,1 - 3 Gew.-%, bevorzugt 0,2 - 2,5 Gew.-%, besonders bevorzugt 0,5 - 2 Gew.-%, außerordentlich bevorzugt 1 - 1,7 Gew.-%, jeweils bezogen auf das Gewicht des Blondierpulvers; wobei der mindestens eine Komplexbildner bevorzugt ausgewählt ist aus Ethylendiamintetraessigsäure (EDTA) und ihren Salzen; N-Hydroxyethylethylendiamintriessigsäure und ihren Salzen; Aminotrimethylenphosphonsäure und ihren Salzen; Diethylentriaminpentaessigsäure und ihren Salzen; Lauroylethylendiamintriessigsäure und ihren Salzen; Nitrilotriessigsäure und ihren Salzen; Iminodibernsteinsäure und ihren Salzen; N-2-Hydroxyethyliminodiessigsäure und ihren Salzen; Ethylenglycol-bis-(beta-aminoethylether)-N,N-tetraessigsäure; Pentanatriumaminotrimethylenphosphonat, sowie Mischungen hiervon.

10. Verfahren zur Aufhellung von keratinischen Fasern, insbesondere menschlichen Haaren, das **dadurch gekennzeichnet ist, dass** ein Blondierpulver nach einem der Ansprüche 1 - 9 mit einer Oxidationszusammensetzung vermischt wird, die, jeweils bezogen auf ihr Gewicht, 50 - 96 Gew.-%, bevorzugt 70 - 93 Gew.-%, besonders bevorzugt 80 - 90 Gew.-%, Wasser und 0,5 - 20 Gew.-% Wasserstoffperoxid enthält und weiterhin mindestens ein pH-Stellmittel in einer solchen Menge enthält, dass die Oxidationszusammensetzung einen pH-Wert im Bereich von 2,5 bis 5,5, gemessen bei 20°C, aufweist, unmittelbar danach auf die keratinhaltigen Fasern aufgebracht, 5 bis 60 Minuten auf den Fasern belassen und anschließend die Fasern mit Wasser gespült und optional mit einem tensidhaltigen Reinigungsmittel ausgewaschen werden,
wobei das Blondierpulver (B) und die Oxidationszusammensetzung (Ox) bevorzugt in einem gewichtsbezogenen Verhältnis (B) : (Ox) von 0,2 - 1, besonders bevorzugt 0,3 - 0,8, weiter bevorzugt 0,4 - 0,7, außerordentlich bevorzugt 0,5 - 0,6, miteinander vermischt werden.

11. Mehrkomponentenverpackungseinheit (Kit-of-Parts) zur Aufhellung von keratinischen Fasern, insbesondere menschlichen Haaren, enthaltend mindestens zwei getrennt voneinander verpackte Komponenten, **dadurch gekennzeichnet, dass**
i) die erste Komponente (I) ein Blondierpulver nach einem der Ansprüche 1 - 9 ist,
ii) die zweite Komponente (II) eine Oxidationszusammensetzung ist, die, jeweils bezogen auf ihr Gewicht, 50 - 96 Gew.-%, bevorzugt 70 - 93 Gew.-%, besonders bevorzugt 80 - 90 Gew.-%, Wasser und 0,5 - 20 Gew.-% Wasserstoffperoxid enthält und einen pH-Wert im Bereich von 2,5 bis 5,5, gemessen bei 20°C, aufweist,
wobei die Komponenten (I) und (II) bevorzugt in einem gewichtsbezogenen Verhältnis (I) : (II) von 0,2 - 1, besonders bevorzugt 0,3 - 0,8, weiter bevorzugt 0,4 - 0,7, außerordentlich bevorzugt 0,5 - 0,6, zueinander vorliegen.

12. Verfahren zur Farbveränderung von keratinischen Fasern, insbesondere menschlichen Haaren, bei dem ein Blondierpulver nach einem der Ansprüche 1 - 9 mit einer Oxidationszusammensetzung (Ox), die, jeweils bezogen auf ihr Gewicht, 50 - 96 Gew.-%, bevorzugt 70 - 93 Gew.-%, besonders bevorzugt 80 - 90 Gew.-%, Wasser und 0,5 - 20 Gew.-% Wasserstoffperoxid und weiterhin mindestens ein pH-Stellmittel in einer solchen Menge enthält, dass die Oxidationszusammensetzung einen pH-Wert im Bereich von 2,5 bis 5,5, gemessen bei 20°C, aufweist,
und zusätzlich mit einer Alkalisierungszusammensetzung (Alk), die Wasser und mindestens ein Alkalisierungsmittel, das ausgewählt ist aus Ammoniak, Alkanolaminen und Mischungen hiervon, enthält und einen pH-Wert im Bereich von 8 - 12, bevorzugt von 9 - 11, besonders bevorzugt von 9,5 - 10,5, aufweist, jeweils gemessen bei 20°C,
vermischt wird,
unmittelbar danach auf die keratinhaltigen Fasern aufgebracht, 5 bis 60 Minuten auf den Fasern belassen und anschließend die Fasern mit Wasser gespült und optional mit einem tensidhaltigen Reinigungsmittel ausgewaschen werden,
wobei das Blondierpulver (B), die Oxidationszusammensetzung (Ox) und die Alkalisierungszusammensetzung (Alk) bevorzugt in einem gewichtsbezogenen Verhältnis (B) : (Ox) : (Alk) von (0,7 - 1,3) : (2 - 3) : (2 - 3), besonders bevorzugt (0,8 - 1,2) ; (2,3 - 2,7) : (2,3 - 2,7), außerordentlich bevorzugt 1:2:2, miteinander vermischt werden.

13. Mehrkomponentenverpackungseinheit (Kit-of-Parts) zur Farbveränderung von keratinischen Fasern, insbesondere menschlichen Haaren, enthaltend mindestens drei getrennt voneinander verpackte Komponenten, **dadurch gekennzeichnet, dass**
i) die erste Komponente (I) ein Blondierpulver nach einem der Ansprüche 1 - 9 ist,
ii) die zweite Komponente (II) eine Oxidationszusammensetzung ist, die, jeweils bezogen auf ihr Gewicht, 50 - 96 Gew.-%, bevorzugt 70 - 93 Gew.-%, besonders bevorzugt 80 - 90 Gew.-%, Wasser und 0,5 - 20 Gew.-% Wasserstoffperoxid enthält und einen pH-Wert im Bereich von 2,5 bis 5,5, gemessen bei 20°C, aufweist,
iii) die dritte Komponente (III) eine Alkalisierungszusammensetzung (Alk) ist, die Wasser und mindestens ein Alkalisierungsmittel, das ausgewählt ist aus Ammoniak, Alkanolaminen und Mischungen hiervon, enthalt und einen pH-Wert im Bereich von 8 - 12, bevorzugt von 9 - 11, besonders bevorzugt von 9,5 - 10,5, aufweist, jeweils gemessen bei 20°C
wobei das Blondierpulver (B), die Oxidationszusammensetzung (Ox) und die Alkalisierungszusammensetzung (Alk) bevorzugt in einem gewichtsbezogenen Verhältnis (B) : (Ox) : (Alk) von (0,7 - 1,3) : (2 - 3) : (2 - 3), besonders bevorzugt (0,8 - 1,2) : (2,3 - 2,7) : (2,3 - 2,7), außerordentlich bevorzugt 1:2:2, zueinander vorliegen.

14. Verwendung einer Kombination aus Bernsteinsäure und/oder mindestens einem Salz dieser Säure sowie Mischungen dieser Verbindungen,
in Kombination mit
Arginin und Lysin oder mindestens einem der Salze dieser Aminosäuren,
in einem Blondierpulver, das mindestens ein Oxidationsmittel, ausgewählt aus Natriumpercarbonaten und anorganischen Salzen einer Peroxoschwefelsäure, und sowie Mischungen hiervon, und weiterhin 0 bis 8 Gew.-% Wasser, bezogen auf das Gewicht des Blondierpulvers, enthält,
zur Verringerung der Schädigung an keratinischen Fasern, insbesondere menschlichen Haaren, die bewirkt wird durch die Behandlung dieser Fasern mit einer Mischung aus dem Blondierpulver und einer Oxidationszusammensetzung, die, jeweils bezogen auf ihr Gewicht, 50 - 96 Gew.-%, bevorzugt 70 - 93 Gew.-%, besonders bevorzugt 80 - 90 Gew.-%, Wasser und 0,5 - 20 Gew.-% Wasserstoffperoxid enthält und einen pH-Wert im Bereich von 2,5 bis 5,5, gemessen bei 20°C, aufweist,
wobei das genannte Blondierpulver bevorzugt ein Blondierpulver nach einem der Ansprüche 2 - 9 ist.

## Claims

1. A bleaching powder, containing
a) at least one oxidizing agent selected from sodium percarbonates and inorganic salts of a peroxysulfuric acid, and mixtures thereof,
b) also at least one dicarboxylic acid having 2 to 10 carbon atoms, selected from succinic acid and/or at least one salt of this acid and mixtures of these compounds,
c) also arginine and lysine or at least one of the salts of these amino acids,
d) and 0 to 8 wt.% water, based on the weight of the bleaching powder.

2. The bleaching powder according to claim 1, **characterized in that** the at least one oxidizing agent a) is contained in a total amount of from 5-85 wt.%, preferably 10-75 wt.%, particularly preferably 15-65 wt.%, and exceptionally preferably 20-55 wt.%, in each case based on the weight of the bleaching powder.

3. The bleaching powder according to claim 1 or 2, **characterized in that** succinic acid and/or at least one salt of these acids is contained in a total amount, converted to the mass of free dicarboxylic acid, of from 0.03-7 wt.%, preferably 0.1-5 wt.%, particularly preferably 0.5-3 wt.%, exceptionally preferably 0.9-1.5 wt.%, in each case based on the weight of the bleaching powder,
dicarboxylic acids having 2 to 10 carbons, selected from succinic acid, malic acid, oxalic acid, malonic acid, adipic acid, pimelic acid, suberic acid, azelaic acid, sebacic acid, maleic acid, fumaric acid, D-tartaric acid, L-tartaric acid, meso-tartaric acid, racemic acid, alpha-ketoglutaric acid, beta-ketoglutaric acid, oxaloacetic acid, and/or the salts of these acids, being contained in a total amount, converted to the mass of free dicarboxylic acid, of from 0.03-7 wt.%, preferably 0.1-5 wt.%, particularly preferably 0.5-3 wt.%, and exceptionally preferably 0.9-1.5 wt.%, in each case based on the weight of the bleaching powder.

4. The bleaching powder according to one of claims 1-3, **characterized in that** arginine and lysine or at least one salt of these amino acids are contained in a total amount, converted to the mass of free amino acid, of from 0.1-7 wt.%, preferably 0.2-5 wt.%, particularly preferably 0.5-2.5 wt.%, exceptionally preferably 1-2 wt.%, in each case based on the weight of the bleaching powder, the amino acids arginine and lysine, possibly histidine, or at least one salt of these amino acids being contained in a total amount, converted to the mass of free amino acid, of from 0.1-7 wt.%, preferably 0.2-5 wt.%, particularly preferably 0.5-2.5 wt.%, exceptionally preferably 1-2 wt.%, in each case based on the weight of the bleaching powder.

5. The bleaching powder according to one of claims 1-4, **characterized in that** succinic acid and/or at least one succinic acid salt are contained in a total amount, converted to the mass of free dicarboxylic acid, of from 0.03-7 wt.%, preferably 0.1-5 wt.%, particularly preferably 0.5-3 wt.%, and exceptionally preferably 0.9-1.5 wt.%, and also arginine and lysine or at least one salt of these amino acids are contained in a total amount, converted to the mass of free amino acid, of from 0.1-7 wt.%, preferably 0.2-5 wt.%, particularly preferably 0.5-2.5 wt.%, and exceptionally preferably 1-2 wt.%, the amount specifications being based on the weight of the bleaching powder.

6. The bleaching powder according to one of claims 1-5, **characterized in that** additionally at least one inorganic alkalizing agent that is solid at 20 °C and 1013 mbar is contained, and is preferably contained in a total amount of from 1-60 wt.%, preferably 5-55 wt.%, particularly preferably 10-50 wt.%, and exceptionally preferably 15-45 wt.%, in each case based on the weight of the bleaching powder.

7. The bleaching powder according to one of claims 1-6, **characterized in that** the at least one oxidizing agent a) is selected from sodium peroxodisulfate, potassium peroxodisulfate, ammonium peroxodisulfate, sodium percarbonate, sodium peroxomonosulfate, potassium peroxomonosulfate, ammonium peroxomonosulfate, and mixtures of these compounds, preferably mixtures of potassium peroxodisulfate and ammonium peroxodisulfate, or mixtures of sodium peroxodisulfate and ammonium peroxodisulfate.

8. The bleaching powder according to one of claims 1-7, **characterized in that** at least one oil is contained in a total amount of from 0.1-15 wt.%, preferably 0.5-10 wt.%, particularly preferably 1-8 wt.%, and exceptionally preferably 2-6 wt.%, in each case based on the weight of the bleaching powder.

9. The bleaching powder according to one of claims 1-8, **characterized in that** at least one complexing agent is contained, preferably in a total amount of from 0.1-3 wt.%, preferably 0.2-2.5 wt.%, particularly preferably 0.5-2 wt.%, and exceptionally preferably 1-1.7 wt.%, in each case based on the weight of the bleaching powder; the at least one complexing agent preferably being selected from ethylenediaminetetraacetic acid (EDTA) and the salts thereof; N-hydroxyethyl ethylenediaminetriacetic acid and the salts thereof; aminotrimethylene phosphonic acid and the salts thereof; diethylenetriaminepentaacetic acid and the salts thereof; lauroyl ethylenediaminetriacetic acid and the salts thereof; nitrilotriacetic acid and the salts thereof; iminodisuccinic acid and the salts thereof; N-2-hydroxyethyl iminodiacetic acid and the salts thereof; ethylene glycol-bis-(beta-aminoethyl ether)-N,N-tetraacetic acid; pentasodium aminotrimethylene phosphonate, and mixtures thereof.

10. A method for lightening keratin fibers, in particular human hair, **characterized in that** a bleaching powder according to one of claims 1 to 9 is mixed with an oxidation composition which contains, in each case based on its weight, 50-96 wt.%, preferably 70-93 wt.%, and particularly preferably 80-90 wt.%, water and 0.5-20 wt.% hydrogen peroxide, and also contains at least one pH adjuster in an amount such that the oxidation composition has a pH in the range of from 2.5 to 5.5, measured at 20 °C, and immediately thereafter is applied to the keratin fibers, left on the fibers for 5 to 60 minutes, and subsequently the fibers are rinsed with water and optionally washed with a surfactant-containing cleansing agent,
the bleaching powder (B) and the oxidation composition (Ox) preferably being mixed together in a weight-based ratio (B):(Ox) of from 0.2-1, particularly preferably 0.3-0.8, more preferably 0.4-0.7, exceptionally preferably 0.5-0.6.

11. A multi-component packaging unit (kit-of-parts) for lightening keratin fibers, in particular human hair, containing at least two components packaged separately from one another, **characterized in that**
i) the first component (I) is a bleaching powder according to one of claims 1 to 9,
ii) the second component (II) is an oxidation composition which contains, in each case based on its weight, 50-96 wt.% preferably 70-93 wt.%, particularly preferably 80-90 wt.%, water and 0.5-20 wt.% hydrogen peroxide and has a pH in the range of from 2.5 to 5.5, measured at 20 °C,
components (I) and (II) preferably being present in a weight-based ratio (I):(II) to one another of from 0,2-1, particularly preferably 0.3-0.8, more preferably 0.4-0.7, exceptionally preferably 0,5-0,6.

12. A method for changing the color of keratin fibers, in particular human hair, in which a bleaching powder according to one of claims 1 to 9 is mixed with an oxidation composition (Ox) which contains, in each case based on its weight, 50-96 wt.%, preferably 70-93 wt.%, particularly preferably 80-90 wt.%, water and 0.5-20 wt.% hydrogen peroxide and also at least one pH adjuster in such an amount that the oxidation composition has a pH in the range of from 2.5 to 5.5, measured at 20 °C,
and additionally with an alkalizing composition (Alk) which contains water and at least one alkalizing agent selected from ammonia, alkanolamines and mixtures thereof, and has a pH in the range of from 8-12, preferably 9-11, particularly preferably 9.5-10.5, in each case measured at 20 °C,
and immediately thereafter is applied to the keratin fibers and left on the fibers for 5 to 60 minutes and subsequently the fibers are rinsed with water and optionally washed with a surfactant-containing cleansing agent,
wherein the bleaching powder (B), the oxidation composition (Ox) and the alkalizing composition (Alk) are preferably mixed together in a weight-based ratio (B):(Ox):(Alk) of from (0.7-1.3):(2-3):(2-3), particularly preferably (0.8-1.2):(2.3-2.7):(2.3-2.7), exceptionally preferably 1:2:2.

13. A multi-component packaging unit (kit-of-parts) for changing the color of keratin fibers, in particular human hair, containing at least three components packaged separately from one another, **characterized in that**
i) the first component (I) is a bleaching powder according to one of claims 1 to 9,
ii) the second component (II) is an oxidation composition which contains, in each case based on its weight, 50-96 wt.% preferably 70-93 wt.%, particularly preferably 80-90 wt.%, water and 0.5-20 wt.% hydrogen peroxide and has a pH in the range of from 2.5 to 5.5, measured at 20 °C,
(iii) the third component (III) is an alkalizing composition (Alk) which contains water and at least one alkalizing agent selected from ammonia, alkanolamines and mixtures thereof and has a pH in the range of from 8-12, preferably 9-11, particularly preferably 9.5-10.5, in each case measured at 20 °C,
the bleaching powder (B), the oxidation composition (Ox) and the alkalizing composition (Alk) preferably being in a weight-based ratio (B):(Ox):(Alk) to one another of from (0.7-1.3):(2-3):(2-3), particularly preferably (0.8-1.2):(2.3-2.7):(2.3-2.7), exceptionally preferably 1:2:2.

14. The use of a combination of succinic acid and/or at least one salt of this acid as well as mixtures of these compounds,
in combination with
arginine and lysine or at least one of the salts of these amino acids,
in a bleaching powder which contains at least one oxidizing agent, selected from sodium percarbonates and inorganic salts of a peroxysulfuric acid, and mixtures thereof, and also 0 to 8 wt.% water, based on the weight of the bleaching powder,
for reducing damage to keratin fibers, in particular human hair, caused by the treatment of these fibers with a mixture of the bleaching powder and an oxidation composition which contains, in each case based on its weight, 50-96 wt.%, preferably 70-93 wt.%, particularly preferably 80-90 wt.%, water and 0.5-20 wt.% hydrogen peroxide and has a pH in the range of from 2.5 to 5.5, measured at 20 °C,
wherein said bleaching powder is preferably a bleaching powder according to one of claims 2-9.

## Revendications

1. Poudre décolorante, contenant
a) au moins un agent oxydant choisi parmi les percarbonates de sodium et les sels inorganiques d'un acide peroxosulfurique, et leurs mélanges,
b) en outre au moins un acide dicarboxylique comportant 2 à 10 atomes de carbone, choisi parmi l'acide succinique et/ou au moins un sel de cet acide et des mélanges de ces composés,
c) en outre de l'arginine et de la lysine ou au moins l'un des sels de ces acides aminés,
d) et 0 à 8 % en poids d'eau, par rapport au poids de la poudre décolorante.

2. Poudre décolorante selon la revendication 1, **caractérisée en ce qu'**elle contient au moins un agent oxydant a) en une quantité totale de 5 à 85 % en poids, de préférence de 10 à 75 % en poids, de manière particulièrement préférée de 15 à 65 % en poids, de manière extrêmement préférée de 20 à 55 % en poids, respectivement par rapport au poids de la poudre décolorante.

3. Poudre décolorante selon la revendication 1 ou 2, **caractérisée en ce qu'**elle contient de l'acide succinique et/ou au moins un sel de ces acides en une quantité totale, convertie en masse d'acide dicarboxylique libre, de 0,03 à 7 % en poids, de préférence de 0,1 à 5 % en poids, de manière particulièrement préférée de 0,5 à 3 % en poids, de manière extrêmement préférée de 0,9 à 1,5 % en poids, respectivement par rapport au poids de la poudre décolorante,
des acides dicarboxyliques comportant 2 à 10 atomes de carbone choisis parmi l'acide succinique, l'acide malique, l'acide oxalique, l'acide malonique, l'acide adipique, l'acide pimélique, l'acide subérique, l'acide azélaïque, l'acide sébacique, l'acide maléique, l'acide fumarique, l'acide D-tartrique, l'acide L-tartrique, l'acide méso-tartrique, l'acide racémique, l'acide alpha-cétoglutarique, l'acide beta-cétoglutarique, l'acide oxaloacétique, et/ou les sels de ces acides, en une quantité totale, convertie en masse d'acide dicarboxylique libre, de 0,03 à 7 % en poids, de préférence de 0,1 à 5 % en poids, de manière particulièrement préférée de 0,5 à 3 % en poids, de manière extrêmement préférée de 0,9 à 1,5 % en poids, respectivement par rapport au poids de la poudre décolorante.

4. Poudre décolorante selon l'une des revendications 1 à 3, **caractérisée en ce qu'**elle contient de l'arginine et de la lysine ou au moins un sel de ces acides aminés en une quantité totale, convertie en masse d'acide aminé libre, de 0,1 à 7 % en poids, de préférence de 0,2 à 5 % en poids, de manière particulièrement préférée de 0,5 à 2,5 % en poids, de manière extrêmement préférée de 1 à 2 % en poids, respectivement par rapport au poids de la poudre décolorante,
les acides aminés arginine et lysine, éventuellement histidine, ou au moins un sel de ces acides aminés, étant contenus en une quantité totale, convertie en masse d'acide aminé libre, de 0,1 à 7 % en poids, de préférence de 0,2 à 5 % en poids, de manière particulièrement préférée de 0,5 à 2,5 % en poids, de manière extrêmement préférée de 1 à 2 % en poids, respectivement par rapport au poids de la poudre décolorante.

5. Poudre décolorante selon l'une des revendications 1 à 4, **caractérisée en ce qu'**elle contient de l'acide succinique et/ou au moins un sel d'acide succinique en une quantité totale, convertie en masse d'acide dicarboxylique libre, de 0,03 à 7 % en poids, de préférence de 0,1 à 5 % en poids, de manière particulièrement préférée de 0,5 à 3 % en poids, de manière extrêmement préférée de 0,9 à 1,5 % en poids, et en outre de l'arginine et de la lysine ou au moins un sel de ces acides aminés en une quantité totale, convertie en masse d'acide aminé libre, de 0,1 à 7 % en poids, de préférence de 0,2 à 5 % en poids, de manière particulièrement préférée de 0,5 à 2,5 % en poids, de manière extrêmement préférée de 1 à 2 % en poids, les quantités indiquées étant basées sur le poids de la poudre décolorante.

6. Poudre décolorante selon l'une des revendications 1 à 5, **caractérisée en ce qu'**elle contient également au moins un agent alcalinisant inorganique solide à 20 °C et à 1013 mbar, lequel est de préférence contenu en une quantité totale de 1 à 60 % en poids, de préférence de 5 à 55 % en poids, de manière particulièrement préférée de 10 à 50 % en poids, de manière extrêmement préférée de 15 à 45 % en poids, respectivement par rapport au poids de la poudre décolorante.

7. Poudre décolorante selon l'une des revendications 1 à 6, **caractérisée en ce que** l'au moins un agent oxydant a) est choisi parmi le peroxodisulfate de sodium, le peroxodisulfate de potassium, le peroxodisulfate d'ammonium, le percarbonate de sodium, le peroxomonosulfate de sodium, le peroxomonosulfate de potassium, le peroxomonosulfate d'ammonium, et des mélanges de ces composés, de préférence des mélanges de peroxodisulfate de potassium et de peroxodisulfate d'ammonium ou des mélanges de peroxodisulfate de sodium et de peroxodisulfate d'ammonium.

8. Poudre décolorante selon l'une des revendications 1 à 7, **caractérisée en ce qu'**elle contient au moins une huile en une quantité totale de 0,1 à 15 % en poids, de préférence de 0,5 à 10 % en poids, de manière particulièrement préférée de 1 à 8 % en poids, de manière extrêmement préférée 2 à 6 % en poids, respectivement par rapport au poids de la poudre décolorante.

9. Poudre décolorante selon l'une des revendications 1 à 8, **caractérisée en ce qu'**elle contient au moins un agent complexant, de préférence en une quantité totale de 0,1 à 3 % en poids, de préférence de 0,2 à 2,5 % en poids, de manière particulièrement préférée de 0,5 à 2 % en poids, de manière extrêmement préférée de 1 à 1,7 % en poids, respectivement par rapport au poids de la poudre décolorante ; l'au moins un agent complexant étant de préférence choisi parmi l'acide éthylènediaminetétraacétique (EDTA) et ses sels ; l'acide N-hydroxyéthyléthylène diamine triacétique et ses sels ; l'acide aminotriméthylène phosphonique et ses sels ; l'acide diéthylènetriaminepentaacétique et ses sels ; l'acide lauroyléthylènediamine triacétique et ses sels; l'acide nitrilotriacétique et ses sels ; l'acide iminodisuccinique et ses sels; l'acide N-2-hydroxyéthyliminodiacétique et ses sels ; l'acide éthylène glycol bis (bêta-aminoéthyl éther)-N,N-tétraacétique ; le phosphonate d'aminotriméthylène pentasodique, ainsi que leurs mélanges.

10. Procédé permettant l'éclaircissement de fibres kératiniques, en particulier de cheveux humains, **caractérisé en ce qu'**une poudre décolorante selon l'une des revendications 1 à 9 est mélangée avec une composition oxydante qui, respectivement par rapport à son poids, contient 50 à 96 % en poids, de préférence 70 à 93 % en poids, de manière particulièrement préférée 80 à 90 % en poids d'eau, et 0,5 à 20 % en poids de peroxyde d'hydrogène, et qui contient en outre au moins un agent d'ajustement de pH en une quantité telle que la composition oxydante présente un pH compris entre 2,5 et 5,5, mesuré à 20 °C, est appliquée immédiatement après sur les fibres kératiniques, est laissée sur les fibres pendant 5 à 60 minutes, puis **en ce que** les fibres sont rincées à l'eau et éventuellement sont lavées avec un agent nettoyant contenant un tensioactif,
la poudre décolorante (B) et la composition oxydante (Ox) étant mélangées de préférence dans un rapport pondéral (B): (Ox) de 0,2 à 1, de manière particulièrement préférée de 0,3 à 0,8, de manière davantage préférée de 0,4 à 0,7, de manière extrêmement préférée de 0,5 à 0,6.

11. Unité de conditionnement multi-constituants (kit de pièces) permettant l'éclaircissement de fibres kératiniques, en particulier de cheveux humains, contenant au moins trois constituants conditionnés séparément les uns des autres, **caractérisée en ce que**
i) le premier constituant (I) est une poudre décolorante selon l'une des revendications 1 à 9,
ii) le deuxième constituant (II) est une composition oxydante qui présente, respectivement par rapport à son poids, 50 à 96 % en poids, de préférence 70 à 93 % en poids, de manière particulièrement préférée 80 à 90 % en poids d'eau et 0,5 à 20 % en poids de peroxyde d'hydrogène, et un pH compris entre 2,5 et 5,5, mesuré à 20 °C,
les constituants (I) et (II) étant présents les uns par rapport aux autres de préférence dans un rapport pondéral (I) : (II) de 0,2 à 1, de manière particulièrement préférée de 0,3 à 0,8, de manière davantage préférée de 0,4 à 0,7, de manière extrêmement préférée de 0,5 à 0,6.

12. Procédé permettant la coloration de fibres kératiniques, en particulier de cheveux humains, selon lequel une poudre décolorante selon l'une des revendications 1 à 9 est mélangée avec une composition oxydante (Ox) qui, respectivement par rapport à son poids, contient 50 à 96 % en poids, de préférence 70 à 93 % en poids, de manière particulièrement préférée 80 à 90 % en poids d'eau et 0,5 à 20 % en poids de peroxyde d'hydrogène, et qui contient en outre au moins un agent d'ajustement de pH en une quantité telle que la composition oxydante présente un pH compris entre 2,5 et 5,5, mesuré à 20 °C,
et également avec une composition alcalinisante (Alk), qui contient de l'eau et au moins un agent alcalinisant choisi parmi l'ammoniac, les alcanolamines et leurs mélanges, et qui présente un pH compris entre 8 et 12, de préférence entre 9 et 11, de manière particulièrement préférée entre 9,5 et 10,5, mesuré respectivement à 20 °C,
est appliquée immédiatement après sur les fibres kératiniques, est laissée sur les fibres pendant 5 à 60 minutes, puis les fibres sont rincées à l'eau et éventuellement lavées avec un agent nettoyant contenant un tensioactif,
la poudre décolorante (B), la composition oxydante (Ox) et la composition alcalinisante (Alk) étant mélangées de préférence dans un rapport pondéral (B) : (Ox): (Alk) de (0,7 - 1,3): (2 - 3): (2 - 3), de manière particulièrement préférée de (0,8 - 1,2): (2,3 - 2,7): (2,3 - 2,7), de manière extrêmement préférée de 1:2:2.

13. Unité de conditionnement multi-constituants (kit de pièces) permettant la coloration de fibres kératiniques, en particulier de cheveux humains, contenant au moins trois constituants conditionnés séparément les uns des autres, **caractérisée en ce que**
i) le premier constituant (I) est une poudre décolorante selon l'une des revendications 1 à 9,
ii) le deuxième constituant (II) est une composition oxydante qui présente, respectivement par rapport à son poids, 50 à 96 % en poids, de préférence 70 à 93 % en poids, de manière particulièrement préférée 80 à 90 % en poids d'eau et 0,5 à 20 % en poids de peroxyde d'hydrogène, et un pH compris entre 2,5 et 5,5, mesuré à 20 °C,
iii) le troisième constituant (III) est une composition alcalinisante (Alk) qui contient de l'eau et au moins un agent alcalinisant choisi parmi l'ammoniac, les alcanolamines et leurs mélanges, et qui présente un pH compris entre 8 et 12, de préférence entre 9 et 11, de manière particulièrement préférée entre 9,5 et 10,5, mesuré respectivement à 20 °C,
la poudre décolorante (B), la composition oxydante (Ox) et la composition alcalinisante (Alk) étant présentes les unes par rapport aux autres de préférence dans un rapport pondéral (B) : (Ox) : (Alk) de (0,7 - 1,3) : (2 - 3) : (2 - 3), de manière particulièrement préférée de (0,8 - 1,2) : (2,3 - 2,7) : (2,3 - 2,7), de manière extrêmement préférée de 1:2:2.

14. Utilisation d'une association d'acide succinique et/ou d'au moins un sel de cet acide ainsi que de mélanges de ces composés,
en association avec
de l'arginine et de la lysine ou au moins l'un des sels de ces acides aminés,
dans une poudre décolorante qui contient au moins un agent oxydant choisi parmi les percarbonates de sodium et les sels inorganiques d'un acide peroxosulfurique, ainsi que leurs mélanges, et qui contient également 0 à 8 % en poids d'eau, par rapport au poids de la poudre décolorante,
pour réduire les dommages aux fibres kératiniques, en particulier aux cheveux humains, qui sont causés par le traitement de ces fibres avec un mélange de la poudre décolorante et d'une composition oxydante, qui, respectivement par rapport à son poids, contient de 50 à 96 % en poids, de préférence de 70 à 93 % en poids, de manière particulièrement préférée de 80 à 90 % en poids d'eau et de 0,5 à 20 % en poids de peroxyde d'hydrogène, et qui présente un pH compris entre 2,5 et 5,5, mesuré à 20 °C,
ladite poudre décolorante étant de préférence une poudre décolorante selon l'une des revendications 2 à 9.
